# EUROPÄISCHE PATENTANMELDUNG

(11) **EP 3 603 742 A1**
(43) Veröffentlichungstag der Anmeldung: **05.02.2020**
(21) Anmeldenummer: 18187092.4
(22) Anmeldetag: 02.08.2018
(51) Int. Cl.: A61N 1/375, A61N 1/39

(54) **IMPLANTAT UND VERFAHREN ZUM HERSTELLEN EINES IMPLANTATS**

(71) Anmelder: BIOTRONIK SE & Co. KG, 12359 Berlin (DE)
(72) Erfinder: Dörr, Thomas, 12437 Berlin (DE); Feese, Ulrich, 14167 Berlin (DE); Klenner, Rolf, 14552 Michendorf (DE); Oertmann, Torsten, 15827 Blankenfelde (DE)
(74) Vertreter: Engelhardt, Björn

(57) **Zusammenfassung**

Die Offenbarung betrifft ein Implantat mit einer Elektrodenanschlussvorrichtung und einem Gehäuse, wobei an der Elektrodenanschlussvorrichtung ein Deckel zum Verschluss des Gehäuses gebildet ist. Des Weiteren ist ein Verfahren zum Herstellen eines Implantats offenbart.

## Beschreibung

Die Offenbarung betrifft ein Implantat und ein Verfahren zum Herstellen eines Implantats.

### Hintergrund

Ein Implantat, wie ein Herzschrittmacher oder ein Defibrillator, enthält unter anderem ein Elektronikmodul mit Chips, eine Batterie zur Energieversorgung und eine Durchführung zu einem Header, an welchem eine oder mehrere Elektroden angeschlossen sein können. Derzeitig bekannte Kontaktierungen zwischen dem Elektronikmodul, der Batterie und der Durchführung werden üblicherweise mit angelöteten oder angeschweißten Kontaktierungsbändern realisiert. In alternativen Ausführungsformen werden gebogene Batteriepins in Steckkontaktierungen angeschlossen.

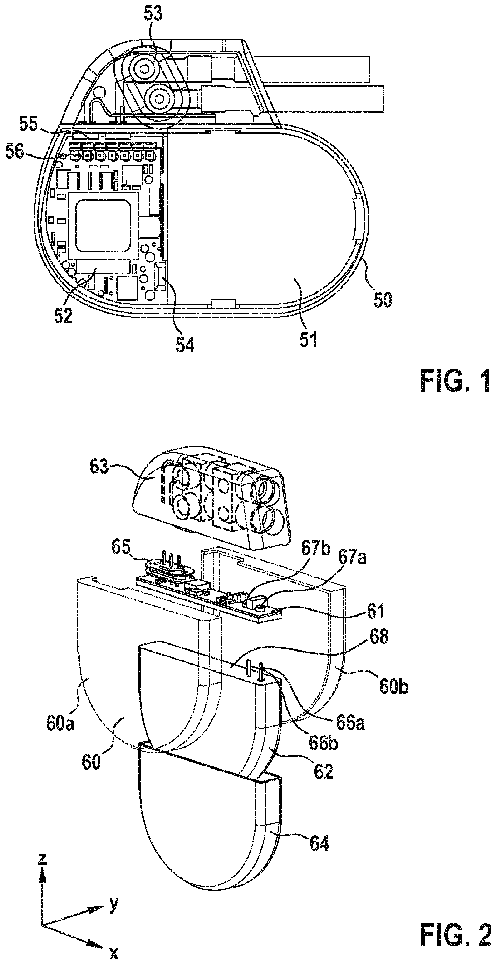

In Fig. 1 ist ein Implantat dargestellt, wie es aus dem Stand der Technik bekannt ist. Das Implantat umfasst ein Gehäuse 50, in dem eine Batterie 51 und ein Elektronikmodul 52 angeordnet sind. An dem Gehäuse 50 ist eine Elektrodenanschlussvorrichtung (Header) 53 angeordnet. Ein erster elektrischer Kontakt 54 ist zwischen dem Elektronikmodul 52 und der Batterie 51 gebildet. Ein zweiter elektrischer Kontakt 56 ist zwischen dem Elektronikmodul 52 und einer Durchführung 55 gebildet. Die Durchführung 55 führt aus dem Gehäuse 50 heraus und stellt eine elektrische Verbindung zwischen dem Elektronikmodul 52 und der Elektrodenanschlussvorrichtung 53 her. Die Elemente des Implantats (Elektrodenanschlussvorrichtung 53, Durchführung 55, Batterie 51 und Elektronikmodul 52) sind flach nebeneinander aneinandergereiht. Dies führt dazu, dass jede elektrische Verbindung zwischen den Elementen (insbesondere der erste elektrische Kontakt 54 und der zweite elektrische Kontakt 56) einen Winkel von 90° durchläuft und damit aufwändige und kostspielige Aufbauprozesse erfordert. Die Herstellung des in Fig. 1 gezeigten Implantats erfordert komplexe Fertigungstechnologie und ist schwierig zu automatisieren.

Das Dokument US 9,737,721 B2 offenbart ein Implantat zur Stimulation des Rückenmarks. Das Implantat umfasst ein Gehäuse, in welchem eine Batterie und ein Elektronikmodul angeordnet sind. Ein auf der Batterie angeordneter Stützrahmen nimmt das Elektronikmodul und eine Kommunikationsspule auf. Das Elektronikmodul ist hierbei senkrecht zur Batterie angeordnet.

Das Dokument US 7,647,110 B2 beschreibt ein modulares Implantat. Verschiedene Konnektormodule, Elektronikmodule und Batteriemodule können miteinander kombiniert werden, um unterschiedliche Funktionen zu realisieren.

Das Dokument EP 2 493 557 B1 offenbart einen modularen Header für ein Implantat. Der Header ist aus mehreren Modulen aufgebaut, die miteinander verbunden sind. Mit der Anzahl der Module kann eine Länge des Headers angepasst werden.

Das Dokument US 2018/0054034 A1 offenbart einen modularen Konnektor, dessen Länge mittels der Anzahl der eingesetzten Module angepasst werden kann.

Das Dokument US 9,713,717 B2 offenbart ein Implantat mit einem Elektronikmodul, das auf einem Substrat gebildet ist. Einige Komponenten des Elektronikmoduls, wie Filterkondensatoren oder Sperrkondensatoren, sind in das Substrat eingebettet.

### Zusammenfassung

Aufgabe ist es, verbesserte Technologien für Implantate anzugeben. Insbesondere soll die Herstellung eines aktiven medizinischen Implantats vereinfacht werden.

Es sind ein Implantat nach Anspruch 1 und ein Verfahren nach Anspruch 15 offenbart. Weitere Ausführungsformen sind Gegenstand von abhängigen Ansprüchen.

Nach einem Aspekt ist ein Implantat mit einem Gehäuse bereitgestellt. In dem Gehäuse sind ein Energiespeicher und ein Elektronikmodul angeordnet. An dem Gehäuse ist eine Durchführung zu einer Elektrodenanschlussvorrichtung gebildet. Ein erster Kontakt bildet eine elektrische Verbindung zwischen dem Energiespeicher und dem Elektronikmodul. Ein zweiter Kontakt bildet eine elektrische Verbindung zwischen dem Elektronikmodul und der Durchführung. Der erste Kontakt und der zweite Kontakt sind in die gleiche Kontaktrichtung ausgerichtet.

Das Implantat kann ein aktives medizinisches Implantat sein, beispielsweise ein implantierbarer Herzschrittmacher oder ein implantierbarer Kardioverter-Defibrillator (ICD - implantable cardioverter-defibrillator).

Die Elektrodenanschlussvorrichtung kann auch als Anschlusskopf oder als Header bezeichnet werden. Die Elektrodenanschlussvorrichtung kann eingerichtet sein, eine oder mehrere Elektrodenanschlüsse aufnehmen.

Das Elektronikmodul kann Komponenten aufweisen, welche den Betrieb des Implantats gewährleisten, beispielsweise einen Prozessor und einen Speicher.

Der Energiespeicher kann eine Primärzelle, eine Sekundärzelle, einen Kondensator oder eine beliebige Kombination der vorgenannten Elemente umfassen. Der Energiespeicher kann eingerichtet sein, die Komponenten des Elektronikmoduls mit elektrischer Energie zu versorgen. Des Weiteren kann der Energiespeicher eingerichtet sein, elektrische Energie für eine Defibrillation (Schock) bereitzustellen. Der Energiespeicher kann gegenüber dem Gehäuse elektrisch isoliert sein, beispielsweise mittels einer Hülle aus einem elektrisch isolierenden Material (z. B. einem thermoplastischem Kunststoff wie PEEK, PEEK - Polyetheretherketon), einem fest anhaftenden Kunststoffüberzug aus einem elektrisch isolierenden Material, einer ein- oder mehrteiligen Isolierfolie, einer Beschichtung aus einem elektrisch isolierenden Material (z. B. Kunststoff) oder mittels Bekleben des Energiespeichers mit einer Isolierfolie.

Die Durchführung kann eine elektrische Verbindung zwischen der Elektrodenanschlussvorrichtung und dem Elektronikmodul bereitstellen. Die Durchführung kann mehrpolig ausgeführt sein, beispielsweise dreipolig, vierpolig oder fünfpolig.

Das Gehäuse kann ein biokompatibles Material aufweisen oder aus einem biokompatiblen Material (z. B. Titan) bestehen.

Eine gleiche Kontaktrichtung ist gegeben, wenn eine Vorzugsrichtung des ersten Kontakts mit einer Vorzugsrichtung des zweiten Kontakts übereinstimmt, also die Kontakte in die gleiche Richtung zeigen. Die Vorzugsrichtung der Kontakte kann sich aus der Geometrie der Kontakte ergeben. Bei einem Stiftkontakt ist die Vorzugsrichtung eine Längserstreckung des Stifts. Bei einem flächigen Kontakt ist die Vorzugsrichtung die Normale der Fläche.

Es kann vorgesehen sein, dass der Energiespeicher, das Elektronikmodul und die Durchführung in einer Stapelrichtung übereinander angeordnet sind, wobei die Stapelrichtung der Kontaktrichtung entspricht. Der Energiespeicher, das Elektronikmodul und die Durchführung werden in einer einzigen Montagerichtung montiert. Das Übereinanderstapeln der Elemente vereinfacht die Montage des Implantats und erleichtert eine Automatisierung des Herstellungsprozesses. Des Weiteren kann die Elektrodenanschlussvorrichtung in der Stapelrichtung auf dem Gehäuse angeordnet sein.

Der erste Kontakt kann zwischen einem ersten flächigen Kontaktelement, das an dem Energiespeicher angeordnet ist, und einem zweiten flächigen Kontaktelement, das an dem Elektronikmodul angeordnet ist, gebildet sein, wobei die Kontaktrichtung die Normale einer Kontaktfläche zwischen dem ersten flächigen Kontaktelement und dem zweiten flächigen Kontaktelement ist. Das erste flächige Kontaktelement und das zweite flächige Kontaktelement können gleich groß sein. Das erste flächige Kontaktelement und das zweite flächige Kontaktelement können die gleiche Form aufweisen, beispielsweise quadratisch, rechteckig, rund oder oval. Das erste flächige Kontaktelement und das zweite flächige Kontaktelement können symmetrisch ausgebildet sein.

Der zweite Kontakt kann zwischen einem dritten flächigen Kontaktelement, das an dem Elektronikmodul angeordnet ist, und einem vierten flächigen Kontaktelement, das an der Durchführung angeordnet ist, gebildet sein, wobei die Kontaktrichtung die Normale einer Kontaktfläche zwischen dem dritten flächigen Kontaktelement und dem vierten flächigen Kontaktelement ist. Das dritte flächige Kontaktelement und das vierte flächige Kontaktelement können gleich groß sein. Das dritte flächige Kontaktelement und das vierte flächige Kontaktelement können die gleiche Form aufweisen, beispielsweise quadratisch, rechteckig, rund oder oval. Das dritte flächige Kontaktelement und das vierte flächige Kontaktelement können symmetrisch ausgebildet sein.

In einer Ausführungsform kann vorgesehen sein, dass der erste Kontakt zwischen einem ersten Stiftelement und einer ersten Stiftaufnahme gebildet ist, wobei eine Längserstreckung des ersten Stiftelements die Kontaktrichtung bestimmt. Das erste Stiftelement kann an dem Energiespeicher angeordnet sein. In diesem Fall ist die erste Stiftaufnahme an dem Elektronikmodul angeordnet. In einer anderen Variante kann das erste Stiftelement an dem Elektronikmodul angeordnet sein und die erste Stiftaufnahme ist an dem Energiespeicher angeordnet. Das erste Stiftelement kann mehrere Stifte umfassen, die parallel zueinander ausgerichtet sind. Es kann vorgesehen sein, dass das erste Stiftelement als ein Paar von Stiften gebildet ist, das beispielsweise an dem Energiespeicher (z. B. als Anode und Kathode des Energiespeichers) angeordnet ist. Die erste Stiftaufnahme ist in diesem Fall als ein Paar von Stiftaufnahmen gebildet, die beispielsweise an dem Elektronikmodul angeordnet sein können.

Des Weiteren kann vorgesehen sein, dass der zweite Kontakt zwischen einem zweiten Stiftelement und einer zweiten Stiftaufnahme gebildet ist, wobei eine Längserstreckung des zweiten Stiftelements die Kontaktrichtung definiert. Das zweite Stiftelement kann an der Durchführung angeordnet sein, wobei die zweite Stiftaufnahme an dem Elektronikmodul angeordnet ist. Alternativ kann das zweite Stiftelement an dem Elektronikmodul angeordnet sein und die zweite Stiftaufnahme ist an der Durchführung angeordnet. Das zweite Stiftelement kann mehrere Stifte umfassen, die parallel zueinander ausgerichtet sind. Die mehreren Stifte können beispielsweise an der Durchführung angeordnet sein (mehrpolige Durchführung). In diesem Fall umfasst die zweite Stiftaufnahme mehrere Stiftaufnahmen, die beispielsweise an dem Elektronikmodul angeordnet sind.

Das erste Stiftelement und das zweite Stiftelement können parallel zueinander angeordnet sein.

Der erste Kontakt und/oder der zweite Kontakt können als Steckkontakt, Klemmkontakt oder Schweißkontakt ausgebildet sein.

Das Elektronikmodul kann an einer Stirnseite des Energiespeichers angeordnet sein. Die Stirnseite ist die der Elektrodenanschlussvorrichtung zugewandte Seite des Energiespeichers. Das Elektronikmodul ist also zwischen der Elektrodenanschlussvorrichtung und dem Energiespeicher angeordnet.

Das Elektronikmodul kann parallel oder senkrecht zur Stirnseite des Energiespeichers angeordnet sein. Das Elektronikmodul kann ein flächiges Substrat aufweisen, auf welchem Komponenten angeordnet sind. Das flächige Substrat kann parallel oder senkrecht zur Stirnseite des Energiespeichers angeordnet sein. Insbesondere eine parallele Anordnung des Elektronikmoduls/des Substrats ermöglicht eine platzsparende Montage. In diesem Fall liegt das Elektronikmodul auf der Stirnseite des Energiespeichers.

Es kann vorgesehen sein, dass das Elektronikmodul in einem Stützrahmen angeordnet ist. Der Stützrahmen kann derart in dem Gehäuse angeordnet sein, dass der Energiespeicher durch den Stützrahmen fixiert ist. Der Stützrahmen kann mit einem Presssitz auf dem Energiespeicher angeordnet sein, so dass der Energiespeicher von dem Stützrahmen gegen das Gehäuse gedrückt wird und hierdurch fixiert ist. Alternativ oder ergänzend kann vorgesehen sein, dass der Stützrahmen derart ausgebildet und in dem Gehäuse angeordnet ist, dass der Stützrahmen eine Relativbewegung zwischen dem Energiespeicher und dem Elektronikmodul reduziert oder verhindert. Insbesondere soll eine Relativbewegung, welche zum Verlust der elektrischen Verbindung zwischen dem Elektronikmodul und dem Energiespeicher führt, verhindert werden. Der Stützrahmen kann einen Kunststoff enthalten oder vollständig aus einem Kunststoff bestehen. Geeignete Kunststoffe sind beispielsweise Polybutylenterephthalat (PBT), Polykarbonat (PC) oder ähnliche Kunststoffe.

Das Gehäuse kann zweiteilig gebildet sein und eine erste Gehäuseschale und eine zweite Gehäuseschale aufweisen. Es kann vorgesehen sein, dass der Energiespeicher zwischen der ersten Gehäuseschale und der zweiten Gehäuseschale fixiert ist. Die erste Gehäuseschale und die zweite Gehäuseschale können symmetrisch (z. B. spiegelsymmetrisch) oder identisch sein. Das zweiteilige Gehäuse kann einen integrierten Schweißschutz (z. B. eine Umbördelung) aufweisen.

In einer Ausführungsform kann das Gehäuse einteilig gebildet sein. Das einteilige Gehäuse kann durch direktes Formen aus einem Grundmaterial hergestellt werden.

Das Gehäuse kann eine Öffnung aufweisen, wobei der Energiespeicher und das Elektronikmodul durch die Öffnung in das Gehäuse einführbar sind. Die Öffnung kann an einer Stirnseite (der Elektrodenanschlussvorrichtung zugewandten Seite) des Gehäuses gebildet sein. Wenn das Gehäuse zweiteilig ist, können die erste Gehäuseschale und die zweite Gehäuseschale miteinander verbunden sein (z. B. verschweißt), so dass die Öffnung an der Stirnseite gebildet ist. Die Öffnung kann in Kontaktrichtung geöffnet sein. In diesem Fall können alle Elemente des Implantats (der Energiespeicher, das Elektronikmodul, die Durchführung, die Elektrodenanschlussvorrichtung und das Gehäuse) in einer einzigen Stapelrichtung montiert werden.

Der Energiespeicher kann am Gehäuse befestigt sein. Beispielsweise kann an der ersten Gehäuseschale und/oder an der zweiten Gehäuseschale ein selbstklebendes Klebepad angebracht sein, an dem/denen der Energiespeicher klebt, wenn die Gehäuseschalen zum Gehäuse verbunden sind. Es kann auch vorgesehen sein, dass der Energiespeicher mittels eines Klebers an der ersten Gehäuseschale und/oder an der zweiten Gehäuseschale geklebt ist. Die Fixierung kann auch durch eine Klemmwirkung zwischen der ersten Gehäuseschale und der zweiten Gehäuseschale realisiert sein.

In dem Gehäuse kann ein Klemmteil angeordnet sein, wobei das Klemmteil eingerichtet ist, den Energiespeicher gegenüber dem Gehäuse zu fixieren. Das Klemmteil kann ein einem unteren Abschnitt des Gehäuses, welcher der Stirnseite des Gehäuses gegenüber liegt, angeordnet sein. Das Klemmteil kann eingerichtet sein, den Energiespeicher zur Fixierung gegen den Stützrahmen zu drücken. Das Klemmteil kann als Feder, als Schweißschutzband oder als festes, raumfüllendes Kunststoffteil ausgeführt sein.

Die Durchführung kann als SMD-Bauelement (SMD - surface-mounted device, oberflächenmontiertes Bauelement) auf dem Elektronikmodul befestigt sein. Ein SMD-Bauelement wird mittels einer oder mehrerer lötfähiger Anschlussflächen direkt auf eine Leiterplatte (z.B. das Substrat des Elektronikmoduls) gelötet. Mit anderen Worten: Die Durchführung ist in SMT-Technologie (SMT - surface-mounting technology, Oberflächenmontage) auf dem Elektronikmodul montiert.

Nach einem weiteren Aspekt ist ein Verfahren zur Montage eines Implantats offenbart. Das Verfahren umfasst folgende Schritte: Bereitstellen eines Energiespeichers, Bereitstellen eines Elektronikmoduls, Bereitstellen einer Durchführung, Anordnen des Elektronikmoduls auf dem Energiespeicher und Anordnen der Durchführung auf dem Elektronikmodul. Hierbei werden die Durchführung, das Elektronikmodul und der Energiespeicher entlang einer gemeinsamen Montagerichtung aufeinander angeordnet. Insbesondere kann das Elektronikmodul auf einer Stirnseite des Energiespeichers angeordnet werden.

Auf die Reihenfolge des Anordnens kommt es nicht an. Es kann zuerst das Elektronikmodul auf dem Energiespeicher angeordnet werden und anschließend die Durchführung auf dem Elektronikmodul. Es kann aber auch zuerst die Durchführung auf dem Elektronikmodul angeordnet werden und anschließend wird das Elektronikmodul mit der Durchführung auf dem Energiespeicher angeordnet.

Beim Anordnen des Elektronikmoduls auf dem Energiespeicher kann eine elektrische Verbindung zwischen dem Energiespeicher und dem Elektronikmodul mit einem ersten Kontakt gebildet werden. Beim Anordnen der Durchführung auf dem Elektronikmodul kann eine elektrische Verbindung zwischen dem Elektronikmodul und der Durchführung mit einem zweiten Kontakt gebildet werden. Der erste Kontakt und der zweite Kontakt können in die gleiche Kontaktrichtung ausgerichtet sein.

Das Verfahren kann weiterhin die folgenden Schritte umfassen: Anordnen des Energiespeichers mit dem Elektronikmodul und der Durchführung in einem Gehäuse und Verschließen des Gehäuses.

Das Verfahren kann des Weiteren die folgenden Schritte umfassen: Anordnen einer Elektrodenanschlussvorrichtung auf dem Gehäuse und Verbinden der Elektrodenanschlussvorrichtung mit der Durchführung.

Nach einem weiteren Aspekt ist ein Implantat mit einem Elektronikmodul und einem Energiespeicher bereitgestellt, wobei das Volumen des Elektronikmoduls kleiner als 25% des Volumens des Energiespeichers ist. Bevorzugt ist das Volumen des Elektronikmoduls kleiner als 20% des Volumens des Energiespeichers. Weiter bevorzugt ist das Volumen des Elektronikmoduls kleiner als 16% des Volumens des Energiespeichers. In einer Ausführungsform beträgt das Volumen des Energiespeichers 3,06 cm³ und das Volumen das Elektronikmoduls beträgt 0,46 cm³.

Die Elemente des Implantats sind dreidimensionale Objekte, die jeweils eine Länge, eine Breite und eine Höhe aufweisen. Die Maße der Objekte werden immer in der gleichen Richtung bestimmt. Die Länge des Elektronikmoduls wird in die gleiche Richtung wie die Länge der Elektrodenanschlussvorrichtung und die Länge der Batterie bestimmt. Die Breite des Elektronikmoduls wird in die gleiche Richtung wie die Breite der Elektrodenanschlussvorrichtung und die Breite der Batterie bestimmt. Die Höhe des Elektronikmoduls wird in die gleiche Richtung wie die Höhe der Elektrodenanschlussvorrichtung und die Höhe der Batterie bestimmt. In der linken unteren Ecke von Fig. 2 ist zur Veranschaulichung ein Koordinatensystem eingezeichnet. Die x-Richtung entspricht der Länge, die y-Richtung gibt die Breite an und die z-Richtung entspricht der Höhe.

Das Volumen des Energiespeichers ist das tatsächliche Volumen des Elements.

Als Volumen des Elektronikmoduls wird das Volumen einer Einhüllenden um das Elektronikmodul angesehen, wobei die Grundfläche der Einhüllenden der Fläche des Elektronikmoduls entspricht und die Höhe der Einhüllenden der Höhe der höchsten Komponente auf dem Elektronikmodul entspricht. Wenn das Elektronikmodul eine rechteckige Grundfläche hat, wird das Volumen also durch einen Quader angegeben, wobei die Grundfläche des Quaders der Grundfläche des Elektronikmoduls (Produkt aus der Länge und der Breite) entspricht. Die Höhe des Quaders entspricht der Höhe der höchsten Komponente auf dem Elektronikmodul. Wenn das Elektronikmodul als flächiges Substrat ausgeführt ist, können auf einer Seite des Substrats Komponenten angeordnet sein. In diesem Fall greift die obige Definition für das Volumen. Es kann auch vorgesehen sein, dass auf beiden Seiten des Substrats Komponenten angeordnet sind. In diesem Fall entspricht die Höhe des Elektronikmoduls der Summe der Höhen der jeweils höchsten Komponente auf den beiden Seiten des Substrats.

Das Verhältnis der Länge des Elektronikmoduls zu der Breite des Elektronikmoduls kann 4:1 oder mehr betragen, bevorzugt 5:1 oder mehr, weiter bevorzugt 6:1 oder mehr. In dieser Ausführungsform hat das Elektronikmodul eine schmale Bauform, was an Anordnen des Elektronikmoduls auf der Stirnseite des Energiespeichers erleichtern kann. In einer Ausführungsform hat das Elektronikmodul eine Länge von mehr als 30 mm und eine Breite von weniger als 5,2 mm.

Es kann vorgesehen sein, dass die Breite des Elektronikmoduls kleiner oder gleich der Breite des Energiespeichers ist.

Die Länge des Elektronikmoduls kann kleiner oder gleich der Länge des Energiespeichers sein.

Wie oben schon erläutert, kann das Implantat eine Elektrodenanschlussvorrichtung aufweisen, wobei die Länge des Elektronikmoduls kleiner oder gleich der Länge der Elektrodenanschlussvorrichtung ist und/oder wobei die Breite des Elektronikmoduls kleiner oder gleich der Breite der Elektrodenanschlussvorrichtung ist. Es kann auch vorgesehen sein, dass die Länge des Energiespeichers kleiner oder gleich der Länge der Elektrodenanschlussvorrichtung ist und/oder dass die Breite des Energiespeichers kleiner oder gleich der Breite der Elektrodenanschlussvorrichtung ist.

Das Elektronikmodul kann ein Substrat aufweisen, auf welchem mehrere Komponenten angeordnet sind, wobei die Fläche des Substrats kleiner oder gleich der Fläche der Stirnseite des Energiespeichers ist.

Es kann vorgesehen sein, dass einige der mehreren Komponenten eine minimale Strukturgröße von F ≤ 90 nm aufweisen. Alternativ oder ergänzend kann vorgesehen sein, dass einige (oder andere) der mehreren Komponente eine minimale Strukturgröße von F ≤ 65 nm, bevorzugt F ≤ 55 nm, aufweisen. Es können alle Komponenten des Elektronikmoduls mit einer einheitlichen Strukturgröße gefertigt sein, beispielsweise F ≤ 90 nm, F ≤ 65 nm oder F ≤ 55 nm. Es kann auch vorgesehen sein, dass die Komponenten des Elektronikmoduls mit verschiedenen der hier genannten Strukturgrößen gefertigt sind.

Wenigstens eine der mehreren Komponenten kann auf einer ersten Seite des Substrats angeordnet sein und wenigstens eine andere der mehreren Komponenten kann auf einer zweiten Seite des Substrats angeordnet sein. Das Substrat kann also einseitig oder zweiseitig bestückt sein.

In einer Ausführungsform kann vorgesehen sein, dass die wenigstens eine Komponente auf der ersten Seite des Substrats und/oder die wenigstens eine andere Komponente auf der zweiten Seite des Substrats mit einem Vergussmittel verkapselt sind.

Es kann vorgesehen sein, dass einige der mehreren Komponenten als SMD-Elemente auf dem Substrat angeordnet sind. Die Komponenten können beispielsweise in einem oder mehreren Ball Grid Array (BGA) und/oder Multi Chip Modul (MCM) Gehäusen und/oder als nackte integrierte Schaltungen (Chips) angeordnet sein. Bei einer einseitigen Bestückung des Substrats sind folgende Anordnungen der Komponenten möglich:
- alle Komponenten sind in einem Ball Grid Array Gehäuse angeordnet,
- alle Komponenten sind in einem MCM Gehäuse angeordnet,
- alle Komponenten sind als Chips angeordnet,
- alle Komponenten sind als SMD-Elemente angeordnet und
- eine oder einige oder keine Komponenten sind in einem oder mehreren BGA Gehäusen angeordnet, eine oder einige oder keine der anderen Komponenten sind in einem oder mehreren MCM Gehäusen angeordnet, eine oder einige oder keine der anderen Komponenten sind als Chips angeordnet und eine oder einige oder keine der anderen Komponenten sind als SMD-Elemente angeordnet.

Bei einer zweiseitigen Bestückung des Substrats können die oben aufgeführten Anordnungen für beide Seiten des Substrats realisiert sein.

Es kann vorgesehen sein, dass einige der mehreren Komponenten auf einer Seite des Substrats nebeneinander oder übereinander angeordnet sind, wobei die Chips/Komponenten jeweils mit dem Substrat verbunden sind und wobei die Komponenten mit einem Vergussmittel verkapselt sind. Die Verbindung der Chips/Komponenten mit dem Substrat kann als Wire-Bonds, Flip Chip Bumps oder Flip-Chip-Lötballen-Verbindung ausgeführt sein. Das Vergussmittel kann das Substrat teilweise bedecken. Das Vergussmittel kann sich in einer Ausführungsform entlang einer Kante des Substrats erstrecken. Bevorzugt bedeckt das Vergussmittel vollständig die Seite des Substrats, auf welcher die Komponenten angeordnet sind.

Auf einem als Substrat dienenden Paneel können die Komponenten gitterförmig angeordnet werden, derart, dass jede Gitterzelle alle für ein Elektronikmodul erforderlichen Einheiten/Chips aufweist. Jede(r) Chip/Komponente wird an das Substrat gebondet, um die elektrischen Verbindungen herzustellen. Anschließend wird das Paneel mit einem Vergussmittel verkapselt (Overmoulding). Nachdem das Paneel mit dem Vergussmittel bedeckt ist, werden die einzelnen Elektronikmodule aus dem Paneel gesägt. Vorteilhafterweise ist die Länge der Vergussfläche auf Paneel ein ganzzahliges Vielfaches der Länge des Elektronikmoduls und/oder die Breite der Vergussbereiches auf dem Paneel ist ein ganzzahliges Vielfaches der Breite des Elektronikmoduls. Hierdurch wird die Vergussfläche des Pannels optimal ausgenutzt. Auf einer anderen Seite des Paneels/Substrats können weitere Komponenten angeordnet sein, beispielsweise als SMD-Elemente, in Chips und/oder in Ball Grid Array Gehäusen.

In dem Vergussmittel können Löcher für einen Anschlusskontakt für den Energiespeicher und/oder für einen weiteren Anschlusskontakt für eine Durchführung gebildet sein.

Das Elektronikmodul kann als ein Multi-Chip-Modul gebildet sein. Ein Multi-Chip-Modul (MCM) besteht aus mehreren einzelnen Mikrochips, die in einem gemeinsamen Gehäuse planar (nebeneinander) oder übereinander untergebracht sind und nach außen wie ein Chip aussehen, so funktionieren und eingesetzt werden.

Die Durchführung zu der Elektrodenanschlussvorrichtung kann als
- integrale Komponente des Elektronikmoduls,
- SMD-Bauelement auf dem Elektronikmodul oder
- Steckverbindung auf dem Elektronikmodul
gebildet sein.

Gemäß einem weiteren Aspekt ist Implantat mit einem Elektronikmodul und einer elektronischen Komponente bereitgestellt, wobei eine elektrische Verbindung zwischen dem Elektronikmodul und der elektronischen Komponente mit einer geraden Steckverbindung gebildet ist.

Das Implantat kann eine weitere elektronische Komponente aufweisen, wobei eine elektrische Verbindung zwischen dem Elektronikmodul und der weiteren elektronischen Komponente mit einer weiteren geraden Steckverbindung gebildet ist und wobei die gerade Steckverbindung und die weitere gerade Steckverbindung in die gleiche Richtung orientiert sind. Die hier offenbarten Merkmale für die gerade Steckverbindung gelten analog für die weitere gerade Steckverbindung. Ebenso gelten die Ausführungen zu der elektronischen Komponente analog für die weitere elektronische Komponente.

Die elektrische Verbindung kann ganz oder teilweise als Steckkontakt derart ausgebildet sein, dass ein Kontaktpin (oder mehrere Kontaktpins) der elektronischen Komponente direkt in eine Steckeraufnahme des Elektronikmoduls auf- oder durchgesteckt werden kann. Die gerade Steckverbindung ist frei von einem Adapter (z.B. einem Verdrahtungsband); des Weiteren ist eine Abwinkelung des Pins nicht notwendig.

Die elektronische Komponente oder die weitere elektronische Komponente können eine Durchführung oder ein Energiespeicher sein. Es können mehrere elektronische Komponenten vorgesehen sein, wobei für jede elektronische Komponente eine elektrische Verbindung mit dem Elektronikmodul mit einer geraden Steckverbindung gebildet ist. Das Elektronikmodul und die elektronische(n) Komponente(n) können in einem Gehäuse angeordnet sein.

In einer Ausführungsform kann ein weiteres Elektronikmodul auf dem Elektronikmodul angeordnet sein. Das weitere Elektronikmodul kann mittels einer geraden Steckverbindung mit dem Elektronikmodul verbunden sein. Es kann ein Stapel von mehreren Elektronikmodulen gebildet sein, wobei die mehreren Elektronikmodule jeweils mit einer geraden Steckverbindung miteinander verbunden sind.

Die elektronische Komponente kann ein gerades Stiftelement aufweisen, wobei das Elektronikmodul eine Stiftaufnahme aufweist, und wobei das Stiftelement in der Stiftaufnahme angeordnet ist, um die elektrische Verbindung zu bilden.

Alternativ kann das Elektronikmodul ein gerades Stiftelement aufweisen, wobei die elektronische Komponente eine Stiftaufnahme aufweist, und wobei das Stiftelement in der Stiftaufnahme angeordnet ist, um die elektrische Verbindung zu bilden.

Wie bereits ausgeführt kann das gerade Stiftelement mehrere Stifte umfassen. Die mehreren Stifte können parallel zueinander angeordnet sein. In diesem Fall sind mehrere Stiftaufnahmen vorgesehen, wobei jeder der mehreren Stifte jeweils einer der mehreren Stiftaufnahmen zugeordnet ist.

Die Stiftaufnahme kann ringförmig sein. Die Stiftaufnahme kann als Scheibe ausgeführt sein. Die Stiftaufnahme kann auf das Elektronikmodul oder auf die elektronische Komponente gelötet sein. Das Stiftelement kann in der Stiftaufnahme verschweißt sein.

Die Stiftaufnahme kann mit einer der folgenden Befestigungsarten an dem Elektronikmodul befestigt sein: Löten, Kleben, Einbetten, Klemmen und Crimpen. Unter Crimpen versteht man ein Fügeverfahren, bei dem zwei Bauteile durch plastische Verformung miteinander verbunden werden, beispielsweise durch Bördeln, Quetschen, Kräuseln oder Falten. Beim Einbetten ist ein Teil der Stiftaufnahme vom Material des Elektronikmoduls umschlossen.

Die Stiftaufnahme kann mit einer der folgenden Befestigungsarten an der elektronischen Komponente befestigt sein: Löten, Kleben, Einbetten, Klemmen und Crimpen.

Es kann vorgesehen sein, dass das Stiftelement ein Federelement aufweist.

Die elektronische Komponente kann ein Energiespeicher, eine Durchführung oder ein Kondensator sein. Die elektronische Komponente kann des Weiteren als Hochspannungskondensator oder als Kondensatorstapel ausgeführt sein. In einer Ausführungsform ist die elektronische Komponente ein Energiespeicher und die weitere elektronische Komponente ist eine Durchführung.

Die Steckverbindung kann als lösbare Verbindung gebildet sein, z. B. als Steckverbindung.

Die Steckverbindung kann als nicht lösbare Verbindung gebildet sein, z. B. als Schweißverbindung oder Lötverbindung.

Die elektrische Verbindung kann mit einer Verbindung ausgewählt aus den folgenden Verbindungsarten gebildet sein: Federkontakt, Schneidklemmkontakt, Lötkontakt, Schweißkontakt, Presspassung und Kleben. Für eine Klebeverbindung kann ein elektrisch leitfähiger Kleber verwendet werden.

Die Steckverbindung kann ausgebildet sein, eine Relativbewegung zwischen der elektronischen Komponente und dem Elektronikmodul auszugleichen, ohne dass die elektrische Verbindung unterbrochen wird. Beispielsweise kann das Stiftelement hinreichend lang und flexibel ausgeführt sein, um eine Relativbewegung der elektronischen Komponente gegenüber dem Elektronikmodul auszugleichen. Alternativ oder ergänzend kann die Steckeraufnahme hinreichend flexibel ausgeführt und/oder montiert sein, um eine Relativbewegung der elektronischen Komponente gegenüber dem Elektronikmodul auszugleichen. Beispielsweise kann das Stiftelement eine Länge aufweisen, die größer ist als eine Höhe der Stiftaufnahme. In diesem Fall ragt das Stiftelement im eingesteckten Zustand über die Stiftaufnahme hinaus, so dass eine Bewegung entlang der Richtung des Stiftelements in einem gewissen Bereich ausgeglichen werden kann.

Gemäß noch einem anderen Aspekt ist ein Verfahren zum Herstellen einer elektrischen Verbindung zwischen einem Elektronikmodul und einer elektronischen Komponente eines Implantats bereitgestellt, wobei die elektronische Komponente und das Elektronikmodul mit einer Relativbewegung aufeinander zu bewegt werden, und die elektrische Verbindung mit einer geraden Steckverbindung zwischen der elektronischen Komponente und dem Elektronikmodul gebildet wird. Die Relativbewegung kann eine gerade Relativbewegung sein. Dies erleichtert die Umsetzung eines automatisierten Prozesses zur Montage des Implantats.

Die Steckverbindung kann als redundante Verbindung ausgeführt sein, um die Zuverlässigkeit zu erhöhen.

Nach noch einem weiteren Aspekt ist ein Implantat mit einer Elektrodenanschlussvorrichtung und einem Gehäuse offenbart, wobei an der Elektrodenanschlussvorrichtung ein Deckel zum Verschluss des Gehäuses gebildet ist.

Der Deckel kann mit dem Gehäuse verschweißt sein.

An dem Deckel kann eine Schweißschutzeinrichtung gebildet sein, beispielsweise in Form einer umlaufenden Umbördelung.

Der Deckel kann aus einem biokompatiblen Material gebildet sein, beispielsweise Titan.

Der Deckel und das Gehäuse können aus dem gleichen Material gebildet sein (z. B. Titan).

Im Deckel kann eine Durchführung gebildet sein, wobei die Durchführung eine elektrische Verbindung zwischen der Elektrodenanschlussvorrichtung und einem in dem Gehäuse angeordneten Elektronikmodul bildet.

Die Durchführung kann mit einer Steckverbindung oder mit einem Federkontakt mit dem Elektronikmodul elektrisch verbunden sein.

Die Elektrodenanschlussvorrichtung kann eine vormontierte Baugruppe aufweisen. Die Baugruppe kann folgende Bauelemente umfassen: eine durchgehende Aufnahmeeinrichtung für einen Stecker, ein erstes Anschlusselement, das in einem vorderen Bereich der Aufnahmeeinrichtung angeordnet ist, wobei das erste Anschlusselement wenigstens zwei ebene Seitenflächen aufweist, und ein zweites Anschlusselement, das in einem hinteren Bereich der Aufnahmeeinrichtung angeordnet ist, wobei das zweite Anschlusselement wenigstens zwei ebene Seitenflächen aufweist.

Nach einem weiteren Aspekt ist eine Baugruppe für eine Elektrodenanschlussvorrichtung eines Implantats bereitgestellt. Die Baugruppe umfasst eine durchgehende Aufhahmeeinrichtung für einen Stecker. Des Weiteren ist ein erstes Anschlusselement vorgesehen, das in einem vorderen Bereich der Aufnahmeeinrichtung angeordnet ist, wobei das erste Anschlusselement wenigstens zwei ebene Seitenflächen aufweist. Schließlich ist ein zweites Anschlusselement vorgesehen, das in einem hinteren Bereich der Aufnahmeeinrichtung angeordnet ist, wobei das zweite Anschlusselement wenigstens zwei ebene Seitenflächen aufweist.

Des Weiteren ist eine Elektrodenanschlussvorrichtung für ein Implantat mit einer hier offenbarten Baugruppe bereitgestellt.

Die Offenbarung umfasst des Weiteren ein Implantat mit einer Elektrodenanschlussvorrichtung und einer Baugruppe.

Die ebenen Seitenflächen ermöglichen eine zumindest teilweise eckige Form und erlauben ein einfaches Greifen der Baugruppe (manuell oder automatisch). Hierdurch kann eine Automatisierung des Herstellungsprozesses ermöglicht werden.

Die Baugruppe kann zumindest abschnittsweise von einem Kunststoff umgeben sein. Beispielsweise kann die Baugruppe von dem Kunststoff abschnittsweise umspritzt sein. Der Kunststoff kann ein Thermoplast sein, beispielsweise Polysulfon. Es kann auch ein biokompatibles Gießharz verwendet werden. Der Kunststoff kann der Baugruppe zusätzliche Stabilität verleihen. Hiermit ist es möglich, die Baugruppe als vorgefertigte Komponente herzustellen, welche anschließend zu einer Elektrodenanschlussvorrichtung eines Implantats verarbeitet wird.

Ein Anschlussbereich des ersten Anschlusselements kann frei von Kunststoff sein. Alternativ oder ergänzend kann ein Anschlussbereich des zweiten Anschlusselements frei von Kunststoff sein.

In dem Kunststoff kann eine erste Führung für einen ersten Leiter zum Anschluss an den Anschlussbereich des ersten Anschlusselements gebildet sein und/oder in dem Kunststoff kann eine zweite Führung für einen zweiten Leiter zum Anschluss an den Anschlussbereich des zweiten Anschlusselements gebildet sein.

Die erste Führung kann benachbart zu dem Anschlussbereich des ersten Anschlusselements gebildet sein und/oder die zweite Führung kann benachbart zu dem Anschlussbereich des zweiten Anschlusselements gebildet sein.

An den Anschlussbereich des ersten Anschlusselements (des zweiten Anschlusselements) kann an erster Leiter (zweiter Leiter) angeschlossen werden, um eine Verbindung von einem in die Aufnahmeeinrichtung eingeführten Stecker zu einem Implantat zu ermöglichen. Der Anschlussbereich des ersten Anschlusselements und/oder der Anschlussbereich des zweiten Anschlusselements können als flächige Elemente ausgeführt sein. Der Anschlussbereich des ersten Anschlusselements und/oder der Anschlussbereich des zweiten Anschlusselements können kreisförmig gebildet sein und beispielsweise einen Durchmesser von 1 bis 5 mm haben. Hierdurch ist eine große Schweißfläche zum Befestigen des ersten Leiters bzw. des zweiten Leiters gegeben. In einer Ausführungsform sind sowohl die erste Führung als auch die zweite Führung benachbart zu den jeweiligen Anschlussbereichen gebildet. Die Führungen ermöglichen einen Anschluss der Leiter an die jeweiligen Anschlussbereiche, ohne einen Kurzschluss zu verursachen.

Es kann vorgesehen sein, dass das erste Anschlusselement und das zweite Anschlusselement zueinander versetzt angeordnet sind. Mit anderen Worten, das erste Anschlusselement und das zweite Anschlusselement liegen auf zwei verschiedenen Ebenen. Die unterschiedliche Anordnung erleichtert es, die Leiter an die Anschlusselemente anzuschließen, ohne dass die Leiter miteinander in Kontakt kommen.

In einer Ausführungsform kann die Baugruppe eine Antenne aufweisen, wobei die Antenne in einem Zwischenbereich, der zwischen dem ersten Anschlusselement und dem zweiten Anschlusselement gebildet ist, einen U-förmigen Verlauf hat. Der Zwischenbereich kann schmaler ausgebildet sein als die angrenzenden Anschlusselemente. Zusammen mit dem U-förmigen Verlauf der Antenne ist hierdurch eine Greifmulde für einen automatischen Greifer gebildet.

An einem hinteren Ende der Aufnahmeeinrichtung kann eine Positioniereinrichtung gebildet sein. Die Positioniereinrichtung kann als abgewinkelte Struktur gebildet sein und beispielsweise einen rechten Winkel zu der Aufnahmeeinrichtung bilden. Die Positioniereinrichtung kann aus dem Kunststoff gebildet sein und beispielsweise mit dem Kunststoffüberzug der Baugruppe einteilig gebildet sein. Die Positioniereinrichtung kann beim Anordnen der Baugruppe in eine Aufnahme an einem Gehäuse des Implantats angeordnet werden, um das Ausrichten der Baugruppe zu erleichtern. Die Positioniereinrichtung kann ein spitz zulaufendes Ende haben.

Die Baugruppe kann eine weitere Aufnahmeeinrichtung für einen weiteren Stecker aufweisen, wobei in einem vorderen Bereich der weiteren Aufnahmeeinrichtung ein drittes Anschlusselement angeordnet ist, und wobei in einem hinteren Bereich der weiteren Aufnahmeeinrichtung ein viertes Anschlusselement angeordnet ist. Für die weitere Aufnahmeeinrichtung gelten die hier offenbarten Ausführungen zu der Aufnahmeeinrichtung analog. Des Weiteren gelten für das dritte Anschlusselement und das vierte Anschlusselement die Ausführungen zum ersten Anschlusselement und zum zweiten Anschlusselement analog.

Nach einem weiteren Aspekt ist ein Verfahren zum Bilden einer Elektrodenanschlussvorrichtung auf einem Implantat offenbart. Das Verfahren umfasst folgende Schritte:
- Bereitstellen einer Baugruppe, mit
   - einer durchgehenden Aufnahmeeinrichtung für einen Stecker,
   - einem ersten Anschlusselement, das in einem vorderen Bereich der Aufnahmeeinrichtung angeordnet ist, wobei das erste Anschlusselement wenigstens zwei ebene Seitenflächen aufweist, und
   - einem zweiten Anschlusselement, das in einem hinteren Bereich der Aufnahmeeinrichtung angeordnet ist, wobei das zweite Anschlusselement wenigstens zwei ebene Seitenflächen aufweist,
- Anordnen und Befestigen eines Federelements in der Aufnahmeeinrichtung,
- Verschließen von Öffnungen der Aufnahmeeinrichtung mit Vergusshilfsmitteln,
- Befestigen eines ersten Leiters an dem ersten Anschlusselement,
- Befestigen eines zweiten Leiters an dem zweiten Anschlusselement,
- Anordnen der Baugruppe auf einem Gehäuse des Implantats,
- Verbinden des ersten Leiters mit einer Durchführung, die an einem Gehäuse gebildet ist,
- Verbinden des zweiten Leiters mit der Durchführung,
- Anordnen der Baugruppe mit dem Gehäuse in einer Gießform,
- Füllen der Gießform mit einem Kunstharz,
- nach Aushärten des Kunstharzes, Entfernen der Vergusshilfsmittel.

Mit dem Verfahren kann ebenfalls eine Elektrodenanschlussvorrichtung auf einem Deckel eines Implantats gebildet werden.

Das Verfahren kann die weiteren Schritte umfassen:
- Anordnen und Befestigen einer Antenne an der Baugruppe,
- Verbinden der Antenne mit der Durchführung,
wobei die weiteren Schritte ausgeführt werden, bevor die Baugruppe auf dem Gehäuse angeordnet wird.

Des Weiteren kann vorgesehen sein, nach dem Aushärten eventuell überstehendes Harz zu entfernen, beispielsweise mittels Schleifen und/oder Polieren.

Die Gießform kann eine Silikonform sein.

Die Durchführung kann einen oder mehrere Steckkontakte (z.B. Stifte) zum Anschluss der Leiter und/oder der Antenne aufweisen.

Das Kunstharz kann ein Epoxidharz sein. Epoxidharze sind Kunstharze, die Epoxidgruppen tragen. Sie sind härtbare Harze (Reaktionsharze), die mit einem Härter und gegebenenfalls weiteren Zusatzstoffen zu einem duroplastischen Kunststoff umgesetzt werden können. Epoxidharze sind Polyether mit zwei endständigen Epoxidgruppen. Die Härtungsmittel sind Reaktionspartner und bilden zusammen mit dem Harz einen makromolekularen Kunststoff.

Das Kunstharz kann direkt auf dem Gehäuse des Implantats oder auf dem Deckel des Implantats haften, so dass ein zusätzliches Haftmittel nicht erforderlich ist. Mit anderen Worten, die Kontaktfläche zwischen dem ausgehärteten Kunstharz und dem Gehäuse / Deckel des Implantats kann frei von einem Haftmittel sein.

Die Elektrodenanschlussvorrichtung kann ein Header für einen implantierbaren Herzschrittmacher oder einen implantierbaren Kardioverter-Defibrillator (ICD - Implantable Cardioverter Defibrillator) sein. In diesem Fall dient die Elektrodenanschlussvorrichtung zur elektrischen Verbindung von einer oder mehreren Elektrodenleitungen mit dem Implantat.

In der Elektrodenanschlussvorrichtung können eine Antenne, eine Ladespule, ein Röntgenmarker, eine Kommunikationsspule und/oder eine Farbmarkierung angeordnet sein.

Ein weiterer Aspekt betrifft ein Verfahren zum Herstellen eines Implantats, mit folgenden Schritten: Bereitstellen eines Gehäuses, Bereitstellen einer Elektrodenanschlussvorrichtung, wobei an der Elektrodenanschlussvorrichtung ein Deckel zum Verschluss des Gehäuses gebildet ist, Anordnen des Deckels auf dem Gehäuse und Verbinden des Deckels mit dem Gehäuse. Die Verbindung zwischen dem Deckel und dem Gehäuse kann als stoffschlüssige Verbindung gebildet sein, beispielsweise mittels Schweißen.

Die hier offenbarten Aspekte zum Implantat und zu der Baugruppe für die Elektrodenanschlussvorrichtung sowie die Aspekte zu den Verfahren können in beliebiger Weise miteinander kombiniert werden, um verschiedene Ausführungsformen des Implantats bzw. der Verfahren zu realisieren. Des Weiteren gelten die Ausführungen zum Implantat und zur Baugruppe analog für die Verfahren und anders herum.

### Beschreibung von Ausführungsbeispielen

Im Folgenden werden beispielhafte Ausführungsformen unter Bezugnahme auf Figuren näher erläutert. Es zeigen:
- Fig. 1: eine schematische Darstellung eines Implantats gemäß dem Stand der Technik,
- Fig. 2: eine Explosionsdarstellung einer Ausführungsform eines erfindungsgemäßen Implantats,
- Fig. 3: eine perspektivische Ansicht eines Teils des Implantats nach Fig. 2,
- Fig. 4: eine Seitenansicht eines Implantats,
- Fig. 5: einen Ausschnitt des Elektronikmoduls,
- Fig. 6: einen weiteren Ausschnitt des Elektronikmoduls,
- Fig. 7: ein Blockschaltbild des erfindungsgemäßen Implantats,
- Fig. 8: eine Seitenansicht des Elektronikmoduls (obere Abbildung von Fig. 8), eine Ansicht des Elektronikmoduls von unten (mittlere Abbildung von Fig. 8) und eine Ansicht des Elektronikmoduls von oben (untere Abbildung von Fig. 8),
- Fig. 9: eine perspektivische Ansicht der Oberseite des Elektronikmoduls (obere Abbildung von Fig. 9) und eine perspektivische Ansicht der Unterseite des Elektronikmoduls (untere Abbildung von Fig. 9),
- Fig. 10A - 10E: Schritte eines Herstellungsprozesses für das Elektronikmodul,
- Fig. 11: eine perspektivische Ansicht einer Ausführungsform einer Baugruppe für eine Elektrodenanschlussvorrichtung,
- Fig. 12: eine Vorderansicht (obere Abbildung von Fig. 12) und eine Hinteransicht (untere Abbildung von Fig. 12) der Baugruppe nach Fig. 11,
- Fig. 13: die Baugruppe nach Fig. 11 und 12 mit Vergusshilfsmitteln,
- Fig. 14: die Baugruppe nach Fig. 11 bis 13 mit einer Antenne und Leitern,
- Fig. 15: die Baugruppe nach Fig. 11 bis 14 auf einem Gehäuse angeordnet,
- Fig. 16: die Baugruppe nach Fig. 11 bis 15 vollständig vergossen (mit Vergusshilfsmitteln),
- Fig. 17: die Baugruppe nach Fig. 11 bis 15 vollständig vergossen (ohne Vergusshilfsmittel) und
- Fig. 18: eine weitere Ausführungsform eines erfindungsgemäßen Implantats.

Fig. 2 zeigt eine Ausführungsform eines Implantats gemäß der Erfindung. Das Implantat umfasst ein zweiteiliges Gehäuse 60 mit einer ersten Gehäuseschale 60a und einer zweiten Gehäuseschale 60b. In dem Gehäuse 60 sind ein Elektronikmodul 61 und ein Energiespeicher 62 (z. B. eine Batterie) angeordnet. Der Energiespeicher 62 ist mittels einer Isolierhülle 64 von dem Gehäuse 60 elektrisch isoliert. Das Elektronikmodul 61 ist auf einer Stirnseite 68 des Energiespeichers 62 angeordnet. An der Stirnseite 68 des Energiespeichers 62 ist ein erstes Stiftelement angeordnet, das zwei parallel zueinander ausgerichtete Stifte 66a, 66b umfasst. Dem ersten Stiftelement ist eine erste Stiftaufnahme zugeordnet, die zwei ringförmige Stiftaufnahmen 67a, 67b umfasst und die an dem Elektronikmodul 61 angeordnet ist. Mittels des ersten Stiftelements 66a, 66b und der ersten Stiftaufnahme 67a, 67b ist eine elektrische Verbindung zwischen dem Energiespeicher 62 und dem Elektronikmodul 61 gebildet. Das Elektronikmodul 61 ist mit einer Durchführung 65 verbunden. Details zu der Verbindung werden weiter unten näher erläutert. Auf dem Gehäuse 60 ist eine Elektrodenanschlussvorrichtung 63 angeordnet, die mittels der Durchführung 65 mit dem Elektronikmodul 61 verbunden ist.

Die Durchführung 65, das Elektronikmodul 61 und der Energiespeicher 62 werden entlang einer Achse montiert (hier entlang der z-Richtung). Die Richtung der Achse ist durch die Richtung der elektrischen Verbindung zwischen der Durchführung 65 und dem Elektronikmodul 61 sowie der elektrischen Verbindung zwischen dem Elektronikmodul 61 und dem Energiespeicher 62 bestimmt.

In der gezeigten Ausführungsform ist das Elektronikmodul 61 parallel zur Stirnseite 68 des Energiespeichers 62 angeordnet. Diese Art der Anordnung nutzt den Raum im Gehäuse sehr effizient. Das Elektronikmodul 61 kann auf die Stirnseite 68 des Energiespeichers 62 aufgesteckt werden und/oder mit der Stirnseite 68 verklebt sein.

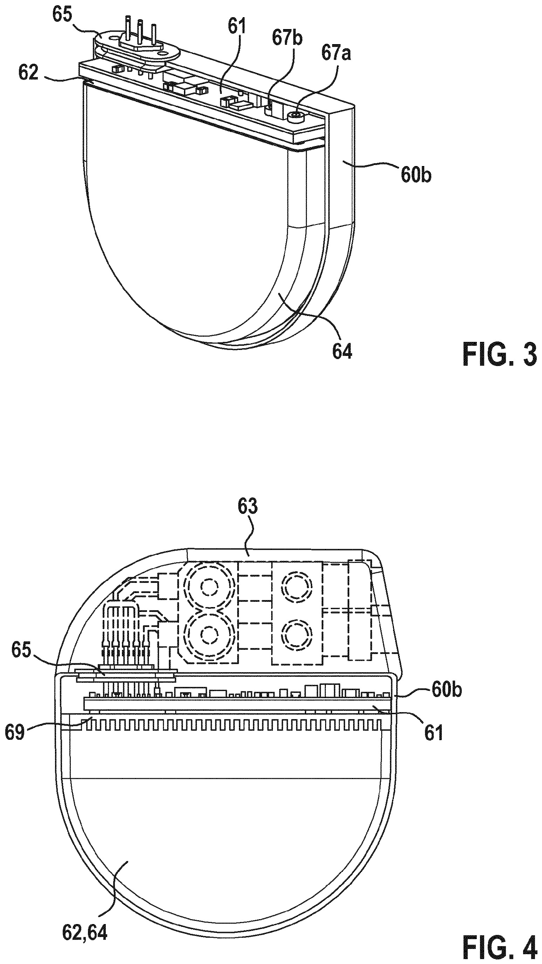

In Fig. 3 sind die Elemente des Implantats teilweise montiert. Das Elektronikmodul 61 ist auf den Energiespeicher 62 aufgesetzt. Des Weiteren ist die Durchführung 65 mit dem Elektronikmodul 61 verbunden. Der Energiespeicher 62 (in der Isolierhülle 64) mit dem Elektronikmodul 61 ist in der zweiten Gehäuseschale 60b angeordnet. Im nächsten Schritt wird die erste Gehäuseschale 60a auf der zweiten Gehäuseschale 60b angeordnet und die Gehäuseschalen 60a, 60b werden miteinander verbunden, z.B. verschweißt (nicht dargestellt). Anschließend wird die Elektrodenanschlussvorrichtung 63 auf das Gehäuse 60 gesetzt und mit der Durchführung 65 verbunden (nicht dargestellt).

Fig. 4 zeigt eine Seitenansicht eines Implantats, das im Wesentlichen dem Implantat nach Fig. 2 entspricht. Gleiche Elemente sind daher mit gleichen Bezugszeichen bezeichnet. Bei der Ausführungsform nach Fig. 4 ist das Elektronikmodul 61 in einem Stützrahmen 69 angeordnet. Der Stützrahmen 69 ist auf der Stirnseite des Energiespeichers 62 angeordnet. Der Stützrahmen 69 dient der Zentrierung des Energiespeichers 62 im Gehäuse 60 und presst diese gegen den Gehäuseboden. Dies verhindert die Weiterleitung von Vibrationen sowie von Druck- und Zugkräften. Der Stützrahmen 69 schützt somit die Komponenten auf dem Elektronikmodul 61 sowie die elektrische Verbindung zwischen dem Energiespeicher 62 und dem Elektronikmodul 61 vor Zerstörung und/oder elektrischem Kontaktverlust.

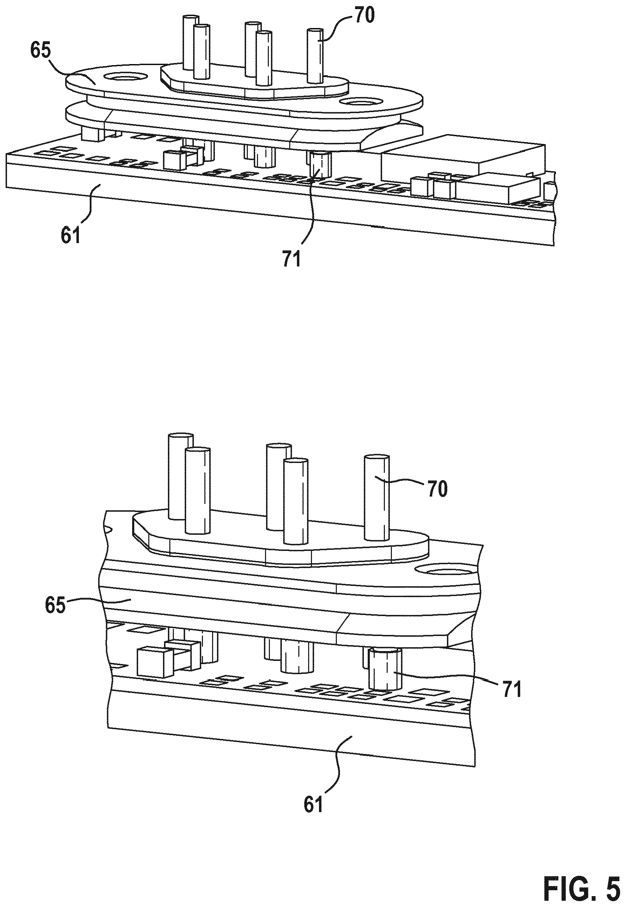

Ein Ausschnitt des Elektronikmoduls 61 ist in Fig. 5 dargestellt (der untere Teil von Fig. 5 zeigt einen vergrößerten Ausschnitt des oberen Teils). Die Durchführung 65 ist als mehrpolige Durchführung mit mehreren Pins 70 gebildet. In der gezeigten Ausführungsform sind fünf Pins 70 an der Durchführung 65 gebildet, eine andere Anzahl von Pins ist jedoch ebenfalls möglich. Jeder Pin 70 steckt in einer Pinaufnahme 71, um mittels der Durchführung 65 eine elektrische Verbindung zwischen dem Elektronikmodul 61 und der Elektrodenanschlussvorrichtung 63 zu bilden.

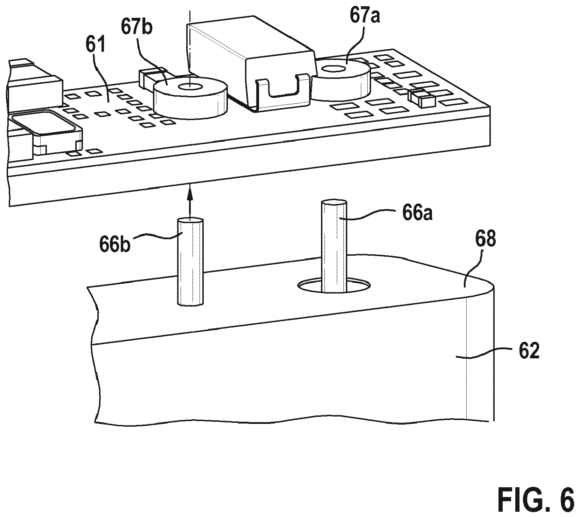

Ein weiterer Ausschnitt des Elektronikmoduls zusammen mit einem Ausschnitt des Energiespeichers 62 ist in Fig. 6 gezeigt. An dem Energiespeicher ist das erste Stiftelement 66a, 66b (z. B. Anode und Kathode der Batterie) gebildet. Auf dem Elektronikmodul 61 ist die erste Stiftaufnahme 67a, 67b angeordnet. Das Elektronikmodul 61 wird mit dem Energiespeicher 62 mittels einer geraden Steckverbindung elektrisch verbunden, indem das erste Stiftelement 66a, 66b in die erste Stiftaufnahme 67a, 67b eingeführt wird. Die Verbindung kann als redundante Verbindung ausgeführt sein, beispielweise indem die erste Stiftaufnahme 67a, 67b jeweils zwei übereinander angeordnete Stiftaufnahmen (zwei übereinander angeordnete Ringe) umfasst (nicht dargestellt).

Die elektrische Verbindung zwischen dem Elektronikmodul 61 und dem Energiespeicher 62 kann mit folgenden Technologien realisiert werden:
- als Zylinder mit Bohrung mit Laserschweißen (vgl. Fig. 6),
- als Federkontakt in einer Steckverbindung und
- als Winkel auf dem Elektronikmodul mit Widerstandsschweißen.

Die Pins 70 der Durchführung 65 sowie das erste Stiftelement 66a, 66b zeigen in die gleiche Richtung (Kontaktrichtung), welche die Montagerichtung für die Elemente bestimmt.

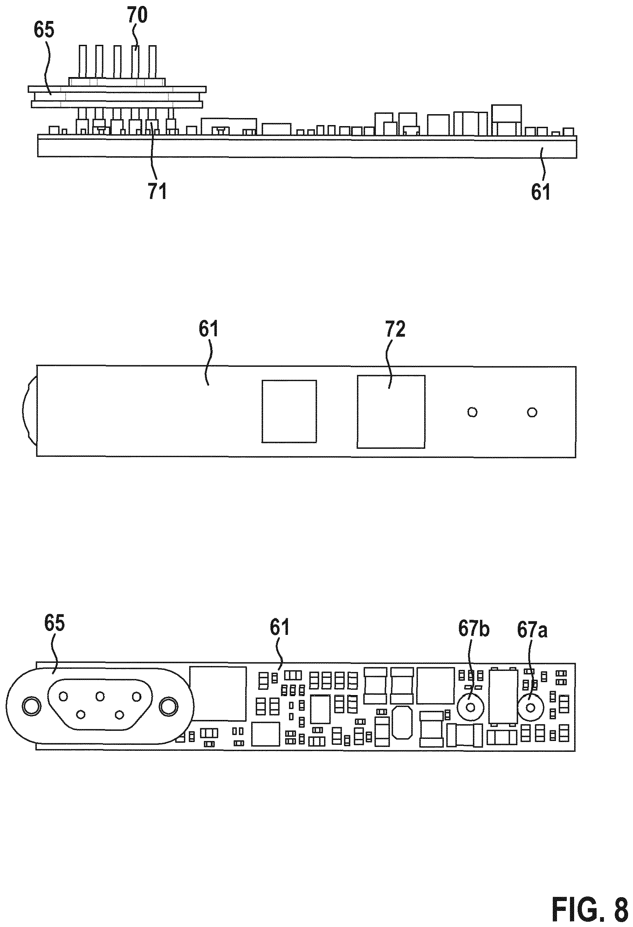

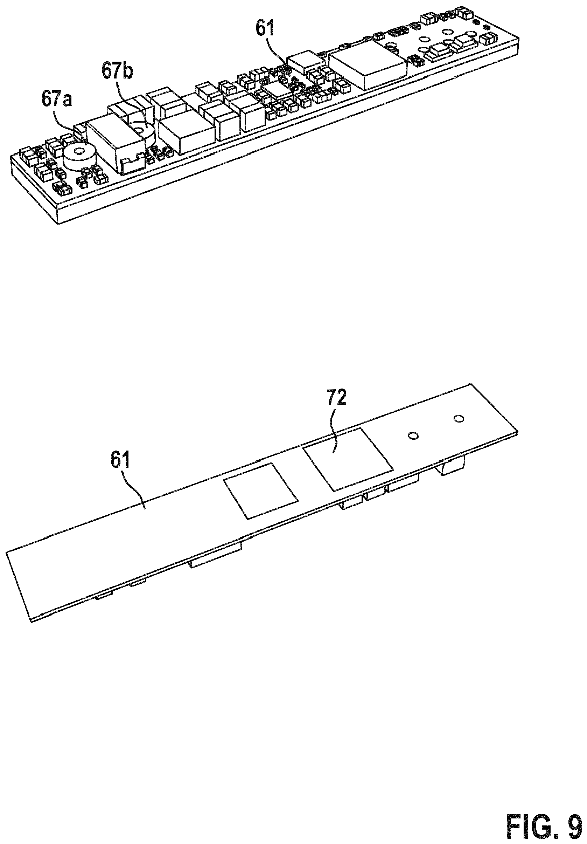

Auf einer Rückseite des Elektronikmoduls ist ein SMD-Bauelement 72 angeordnet (vgl. Fig. 8 und 9).

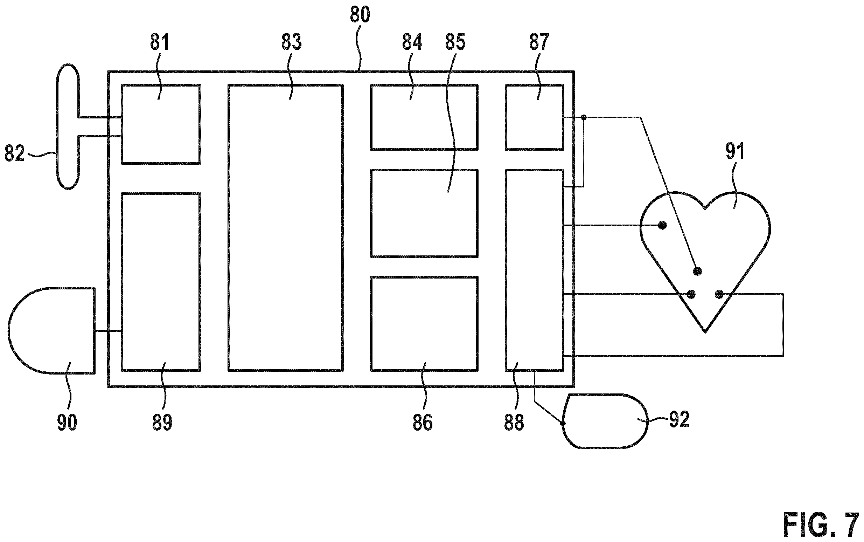

Fig. 7 zeigt ein Blockschaltbild des Implantats. Die Funktionen des Elektronikmoduls sind von dem Rahmen 80 umgeben, die als Einheiten / Chips auf dem Elektronikmodul realisiert sind und im Folgenden näher erläutert werden.

Ein Radio-Transceiver 81 ist mit einer Antenne 82 gekoppelt. Der Radio-Transceiver 81 dient zur Kommunikation mit einem externen Gerät, insbesondere einem Programmiergerät. Hierbei können beispielsweise Messwerte und/oder Parameter des Implantats an das Programmiergerät übertragen werden. Es können auch geänderte Parameter für das Implantat vom Programmiergerät empfangen werden.

Das Elektronikmodul umfasst des Weiteren eine Steuereinheit 83 (Controller). Die Steuereinheit 83 weist einen Prozessor auf, z. B. einen digitalen Signalprozessor (DSP - digital signal processor), einen Speicher wie RAM (RAM - random-access memory, Direktzugriffsspeicher) und/oder ROM (ROM - read only memory, Nur-Lese-Speicher) und eine Zeiteinheit (Timer). Als weitere Funktionen können ein Speicherzugriff, z.B. DMA (DMA - direct memory access, Speicherdirektzugriff) und/oder Netzwerkfunktionen wie MAC (MAC - media access control, Medienzugriffssteuerung) in die Steuereinheit 83 integriert sein.

Als weitere Komponente ist eine Messeinheit 86 (Sensing-Einheit) auf dem Elektronikmodul vorgesehen. Die Messeinheit 86 ist konfiguriert, Messwerte vom Herzen 91 aufzunehmen.

Eine Schrittmachereinheit 85 (Pacing-Einheit) ist konfiguriert, Stimulationsimpulse für das Herz 91 zu erzeugen.

Das Elektronikmodul kann optional eine Schockeinheit 84 (Shocking-Einheit) und eine HV-Einheit 87 (HV - high voltage, Hochspannung) umfassen, insbesondere wenn das Implantat als ICD ausgeführt ist. Die Schockeinheit 84 ist eingerichtet, die HV-Einheit 87 zu steuern. Die HV-Einheit 87 ist konfiguriert, einen Schock abzugeben (Defibrillation), beispielsweise mit einer Spannung von 700 - 800 V.

Es ist eine EMV-Einheit 88 (EMV - Elektromagnetische Verträglichkeit) vorgesehen, die eingerichtet ist, den Einfluss von elektro-magnetischen Feldern zu minimieren oder zu unterdrücken. Die elektro-magnetischen Felder können Störstrahlung, das Feld eines von der HV-Einheit 87 abgegebenen Schocks, das Feld eines von der Schrittmachereinheit 85 abgegebenen Stimulationspulses, das Feld eines externen Schocks, das Feld einer externen Stimulation und Felder anderer externer Quellen (z. B. einer Hochfrequenzmessung) umfassen. Die EMV-Einheit 88 ist mit dem Gehäuse 92 des Implantats gekoppelt.

Das Elektronikmodul ist mit einer Batterie 90 gekoppelt. Eine Energieeinheit 89 des Elektronikmoduls umfasst ein Schaltnetzteil (SMPS - switched-mode power supply) und ist für ein Powermanagement konfiguriert.

Die Funktionen/Einheiten des Elektronikmoduls sind in verschiedenen integrierten Schaltungen, d. h. in auf dem Elektronikmodul montierten Chips, implementiert. Die Abmessungen der Chips ergeben sich durch ihre Komplexität an Funktionen. Je höher die Komplexität, umso größer ist die planare Ausdehnung der Chips. Die Größe der Chips bestimmt maßgeblich die Größe des Elektronikmoduls und auch dessen Ausrichtung im Implantat (parallel zum Energiespeicher). Auch wird die Größe des Elektronikmoduls bestimmt durch die Anzahl der elektrischen Verbindungen der Chips untereinander, der Chips mit den nicht integrierten passiven Komponenten und alle weiteren Verbindungen auf dem Elektronikmodul.

Welche Therapiefunktionen mit welchem Herstellungsprozess miteinander auf einem Chip monolithisch integriert werden können oder wie viele Chips das Elektronikmodul enthält, hängt von ihrem Arbeitsspannungsbereich, ihrer Daten- und Signalkomplexität sowie von ihrem Charakter ab, d.h. ob sie ein analoges, zeitkontinuierliches oder ein digitales, zeitdiskretes oder ein analog-digital, gemischtes Signalverhalten haben. Im Prinzip können alle oben aufgeführten Funktionen monolithisch integriert werden, insbesondere die digitalen Steuerfunktionen, die analog-digital gemischten Sensing Funktionen zur EKG Signalverstärkung und -bewertung (EKG - Elektrokardiogramm), das Pacing zur Stimulationspulserzeugung, das Powermanagement zur optimalen Energieversorgung des Implantates und das Shocking zur Spannungserzeugung und Steuerung des Defibrillationsschockes. Mit den derzeit verwendeten Herstellungsprozessen mit minimalen Strukturgrößen F = 130 nm und F = 180 nm resultieren jedoch Chips, deren Abmessung zu groß für eine vertikale Anordnung im Implantatgehäuse sind und darüber hinaus deren Datenspeicherkapazität (RAM) zu klein ist bzw. mit einem weiteren Speicherchip ergänzt werden muss, um alle erforderten Therapie- und Diagnosefunktionen zu ermöglichen. Für das vorliegende Implantat werden daher einige oder alle Funktionen des Elektronikmoduls mit Chips implementiert, die mit einer minimalen Strukturgröße F ≤ 90 nm, bevorzugt F ≤ 65 nm oder F ≤ 55 nm, hergestellt werden.

Ein Ziel ist es, dass Elektronikmodul derart auszugestalten, dass es Volumen, Form und Größe des Implantates nicht mehr bestimmt (wie im Stand der Technik). Dazu wird mindestens eine der folgenden Regeln angewandt:
1. Das Elektronikmodul wird parallel zur Stirnseite des Energiespeichers angeordnet.
2. Die Länge des Elektronikmoduls ist kleiner oder gleich der Länge der Elektrodenanschlussvorrichtung.
3. Die Breite des Elektronikmoduls ist kleiner oder gleich der Breite des Energiespeichers (oder der Breite des Gehäuses).
4. Die Länge des Energiespeichers ist gleich der Länge der Elektrodenanschlussvorrichtung (maximale Volumenausnutzung).
5. Die Fläche des Elektronikmoduls entspricht der Fläche der Stirnseite des Energiespeichers.
6. Der Volumenbedarf des Elektronikmodules beträgt weniger als 1/4 des Volumens des Energiespeichers (oder weniger als 1/4 des gesamten metallumhüllenden Volumen des Implantates).

Bei Anwendung einer oder mehrerer dieser Regeln entsteht ein streifenförmiges, schmales Elektronikmodul, das mit Komponenten bestückt wird, deren maximale Kantenlänge inklusive ihrer Anschlussverbindungen das Elektronikmodul nicht überragt. Durch verbesserte optischen Abbildungen und Lithographie-Verfahren in der Halbleiterherstellung wird immer mehr Funktionalität pro Siliziumfläche ermöglicht. Eine für die Herstellung des Implantats ausreichende minimale Strukturgröße der Komponenten ist F ≤ 90 nm. Dies ermöglicht es, Analog-, Digital-, Analog-Digital gemischte und Hochspannungsschaltungen zunehmend auf einem Chip monolithisch zu intergieren. Hierdurch wird die Anzahl der Chips und die Anzahl der Verbindungen auf dem Elektronikmodul reduziert, womit die kleinere zur Verfügung stehende Fläche des nun schmalen, streifenförmigen Modules wieder kompensiert wird, um die bekannte funktionale Komplexität zu ermöglichen.

Für die Herstellung der integrierten Schaltungen auf dem Elektronikmodul werden Herstellungsprozesse mit folgenden Merkmalen ausgewählt: Es wird mindestens ein Chip in einem Prozess mit minimaler Strukturgröße F ≤ 90 nm hergestellt. Alternativ wird mindestens ein Chip in einem Prozess mit minimaler Strukturgröße F ≤ 65 nm hergestellt. Alternativ wird mindestens ein Chip in einem Prozess mit minimaler Strukturgröße F ≤ 65 nm hergestellt, auf dem gleichzeitig eine Spannung von ≥ 10 V bezüglich seines Substrates geschaltet werden kann. Alternativ wird mindestens ein Chip in einem Prozess mit minimaler Strukturgröße F ≤ 65 nm hergestellt, auf dem gleichzeitig eine Spannung von ≥ 10 V bezüglich seines Substrates geschaltet werden kann und die SRAM Speicherfunktionen (SRAM - static random-access memory, statisches RAM) des Chips hat eine Kapazität von ≥ 3 Megabit.

Das Elektronikmodul mit dem hier beschrieben Formfaktor eignet sich für eine Montage im Querschnitt des flachen Implantates, insbesondere zwischen dem Energiespeicher und der Elektrodenanschlussvorrichtung. Durch diesen Montageort ergeben sich weitere Merkmale des Elektronikmoduls, die einzeln oder in beliebiger Kombination miteinander realisiert werden können:
- senkrechte Anschlüsse für bedrahtete Komponenten auf der Oberseite und/oder Unterseite Elektronikmoduls,
- Bohrungen zum Auf- oder Durchstecken von Anschlüssen von Komponenten, z. B. Batterieanschlüsse und/oder Headeranschlüsse,
- eine Bohrpassung zur Aufnahme von Schneidhülsen und/oder Klemmhülsen.

Gesägte, gerade Kanten ermöglichen auch eine optimale Fertigung des Elektronikmoduls im Nutzen in einer standardmäßigen Verpackungstechnologie für Ball Grid Array Packages (µBGAs). Beim µBGA werden die Chips auf ein Leiterplattensubstrat (PCB Substrat) montiert, gebondet und mit einer Moldmasse in einem Transferovermoldprozess abgedeckt. Auf der Rückseite des Substrates werden Lötkugeln aufgebracht zur SMD Montage. Die Verpackung findet im Nutzen statt. Dabei hat das Leiterplattensubstrat für alle Chipgrößen immer eine einheitliche Paneelgröße, die nur vom Gussformwerkzeug der Transferovermoldmaschine abhängt. Je nach Chipgröße passen mehr oder weniger viele Chips auf das Panel, die dann nach dem Verguss und dem Bekugeln in ihrer endgültigen Gehäusegröße vom Paneel ausgesägt werden.

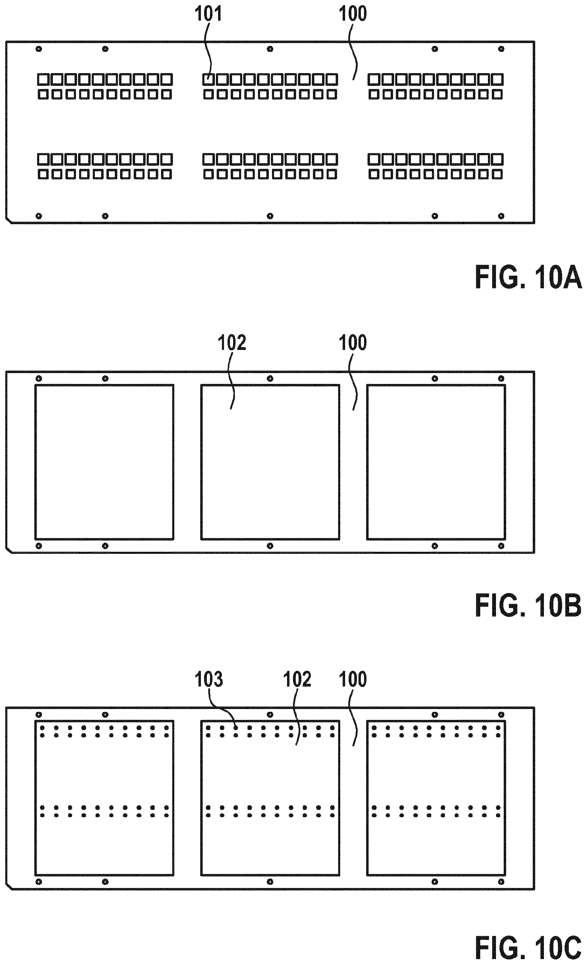

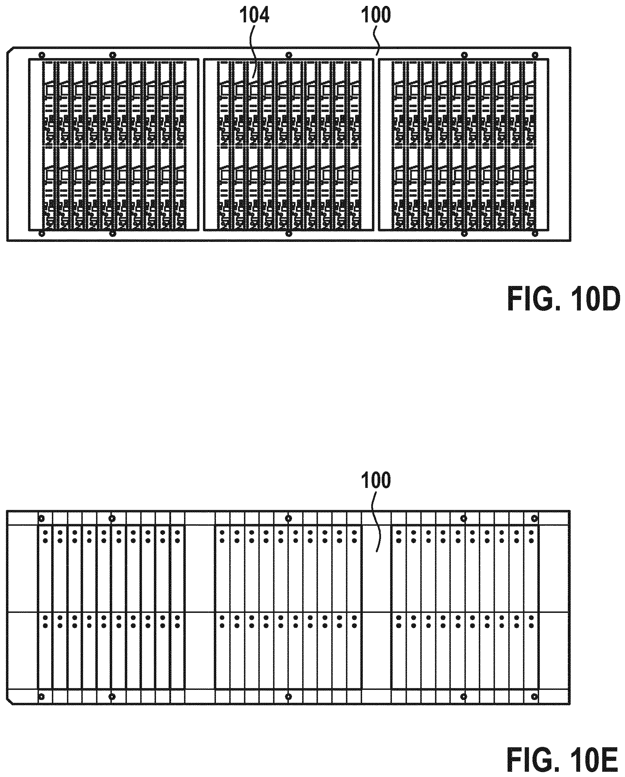

In einer Ausführungsform befinden sich auf einem 205 mm x 70 mm großen Paneel drei quadratische Vergussflächen mit je 56 mm Kantenlänge, in die die Chips montiert und drahtgebondet werden (vgl. Fig. 10A und 10B). Die Anwendung dieses µBGA Verpackungsprozesses auf das Elektronikmodul führt zu weiteren Kennzeichen und Erweiterungen des Verpackungsprozesses, die in den Fig. 10A bis 10E dargestellt sind. Nach dem Transfermolden werden die Löcher oder Passungen der Schneidhülsen und/oder Klemmhülsen gebohrt (vgl. Fig. 10C). Der Verguss dient nicht nur zur Abdeckung der Chips, er ist auch Teil der mechanischen Stabilität des Elektronikmoduls und insbesondere der resultierenden Steckverbindung. Anstelle der Lötkugeln wird das Paneel mit den SMD-Bauelementen der Elektronikmodule im Nutzen bestückt (vgl. Fig. 10D). Die Kantenlängen des Elektronikmoduls erfüllt ein ganzzahliges Teilerverhältnis der Vergussflächenkanten des Paneels abzüglich der Sägeverluste. Die Komponenten des Elektronikmoduls können als ASICs (ASIC - application-specific integrated circuit, anwendungsspezifische integrierte Schaltung) bereitgestellt sein.

Einige Herstellungsschritte sind im Folgenden nochmal zusammengefasst:
- Fig. 10A: ASICs 101 werden im Nutzen auf einer ersten Seite (Vorderseite) des Paneels 100 montiert und gebondet.
- Fig. 10B: Transferovermolding der monierten ASICs 101 mit einem Vergussmittel 102.
- Fig. 10C: Bohren der Löcher 103 für einen Anschluss des Energiespeichers.
- Fig. 10D: Bestückung einer zweiten Seite (Rückseite) des Panels 100 mit SMD-Bauelementen 104.
- Fig. 10E: Aussägen der fertigen Elektronikmodule.

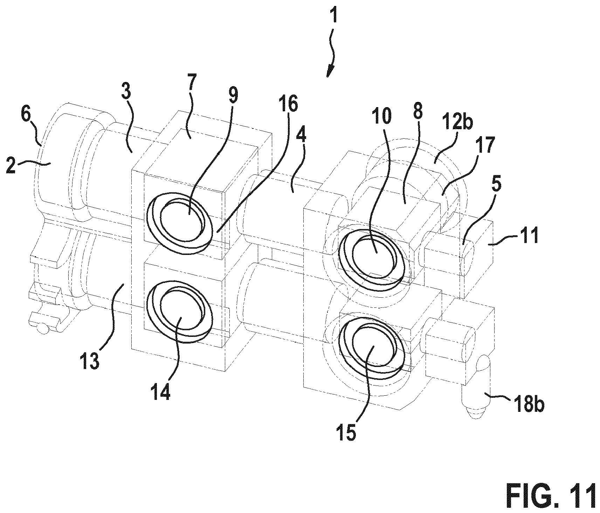

In den Fig. 11 bis 17 sind die einzelnen Schritte zur Montage einer Elektrodenanschlussvorrichtung (Header) auf einem Implantat dargestellt. Die Schritte werden im Folgenden näher erläutert.

Fig. 11 zeigt eine Baugruppe 1 (auch als Headerkern bezeichnet) mit einer ersten Aufnahmeeinrichtung 2 für einen Elektrodenstecker und einer zweiten Aufnahmeeinrichtung 13 für einen weiteren Elektrodenstecker. Die erste Aufnahmeeinrichtung 2 weist eine vordere Öffnung 6 auf, durch welche der Elektrodenstecker eingeführt werden kann. Die erste Aufnahmeeinrichtung 2 weist einen ersten Abschnitt 3, einen zweiten Abschnitt 4 und einen dritten Abschnitt 5 auf. Der Durchmesser des ersten Abschnitts 3 ist größer als der Durchmesser des zweiten Abschnitts 4. Der Durchmesser des zweiten Abschnitts 4 ist wiederum größer als der Durchmesser des dritten Abschnitts 5. Mit anderen Worten: die erste Aufnahmeeinrichtung 2 ist von der vorderen Öffnung 6 zum Ende hin stufenweise verjüngt.

Zwischen dem ersten Abschnitt 3 und dem zweiten Abschnitt 4 (also in einem vorderen Bereich der ersten Aufnahmeeinrichtung 2) ist ein erstes Anschlusselement gebildet. Zwischen dem zweiten Abschnitt 4 und dem dritten Abschnitt 5 (in einem hinteren Bereich der ersten Aufnahmeeinrichtung 2) ist ein zweites Anschlusselement 8 gebildet. Sowohl das erste Anschlusselement 7 als auch das zweite Anschlusselement 8 weisen mindestens zwei ebene Seitenflächen auf. Dies ermöglicht ein einfaches Greifen der Baugruppe 1 während einer Montage und ermöglicht eine Automatisierung der Montageschritte. In der dargestellten Ausführungsform sind das erste Anschlusselement 7 und das zweite Anschlusselement 8 im Wesentlichen quaderförmig gebildet. Das zweite Anschlusselement 8 weist eine abgeschrägte Kante 17 auf, welche zur Materialeinsparung und zur Beachtung der Fließrichtung des Epoxidharzes dient. An einer Rückseite des ersten Anschlusselements 7 ist eine Aussparung 12a in dem Kunststoffüberzug gebildet. An einer Rückseite des zweiten Anschlusselements 8 ist eine hintere Öffnung 12b gebildet.

Die Baugruppe ist zum Teil von einem Kunststoff 11 umgeben. In der dargestellten Ausführungsform ist die Baugruppe teilweise mit Polysulfon umspritzt. In dem Kunststoff 11 sind Aussparungen für eine erste Kontaktfläche 9 und eine zweite Kontaktfläche 10 gebildet. Die erste und zweite Kontaktfläche sind als kreisförmige Flächen gebildet. Benachbart zu der ersten und zweiten Kontaktfläche 9, 10 ist jeweils eine Führung 16 gebildet. Die Führung 16 dient zur Aufnahme eines Anschlusselements (z.B. eines Verdrahtungsbandes). Die Führungen an den Kontaktflächen verhindern, dass Anschlusselemente von verschiedenen Kontaktflächen einander berühren.

Die zweite Aufnahmeeinrichtung 13 ist analog zu der ersten Aufnahmeeinrichtung 2 aufgebaut. Aus Gründen der Übersichtlichkeit sind die Komponenten der zweiten Aufnahmeeinrichtung (eine Öffnung, die drei stufenförmig verjüngenden Abschnitte sowie die zwei Anschlusselemente) nicht mit Bezugszeichen versehen. Die zweite Aufhahmeeinrichtung weist ebenfalls zwei Kontaktflächen (dritte Kontaktfläche 14 und vierte Kontaktfläche 15) für Anschlüsse auf. Benachbart zu den Kontaktflächen sind wiederum Führungen gebildet.

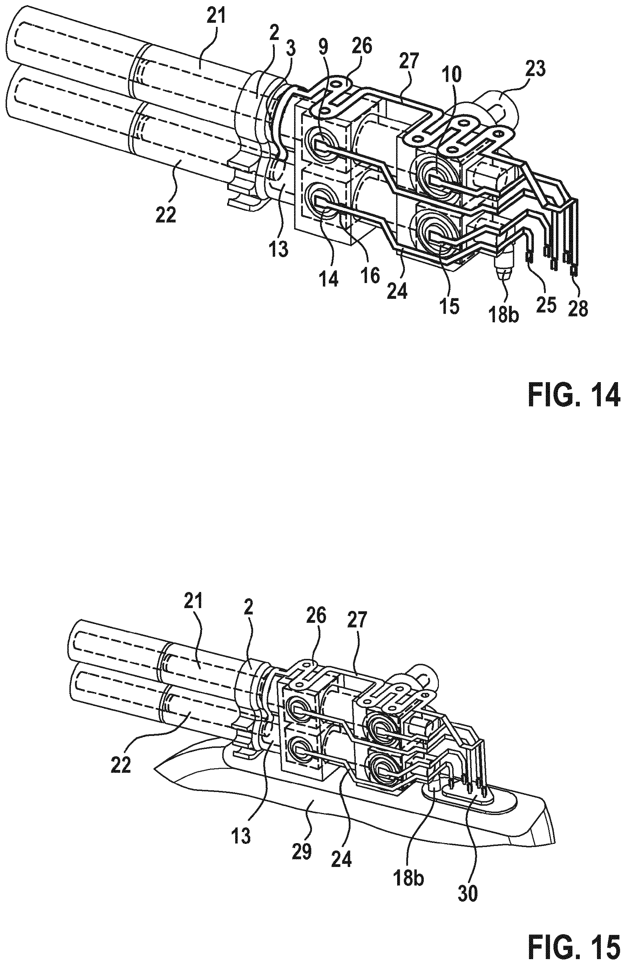

An einem Ende der zweiten Aufnahmeeinrichtung ist eine Positioniereinrichtung 18b gebildet, welche als Zapfen mit einem spitz zulaufenden Ende ausgeführt ist. Beim Montieren der Baugruppe 1 auf einem Gehäuse 29 (vgl. Fig. 15) kann das spitze Ende der Positioniereinrichtung 18b in eine Aufnahme des Gehäuses eingesetzt werden, um das passgenaue Anordnen der Baugruppe auf dem Gehäuse zu erleichtern. Die Baugruppe kann jedoch auch ohne Positioniereinrichtung 18b ausgeführt sein.

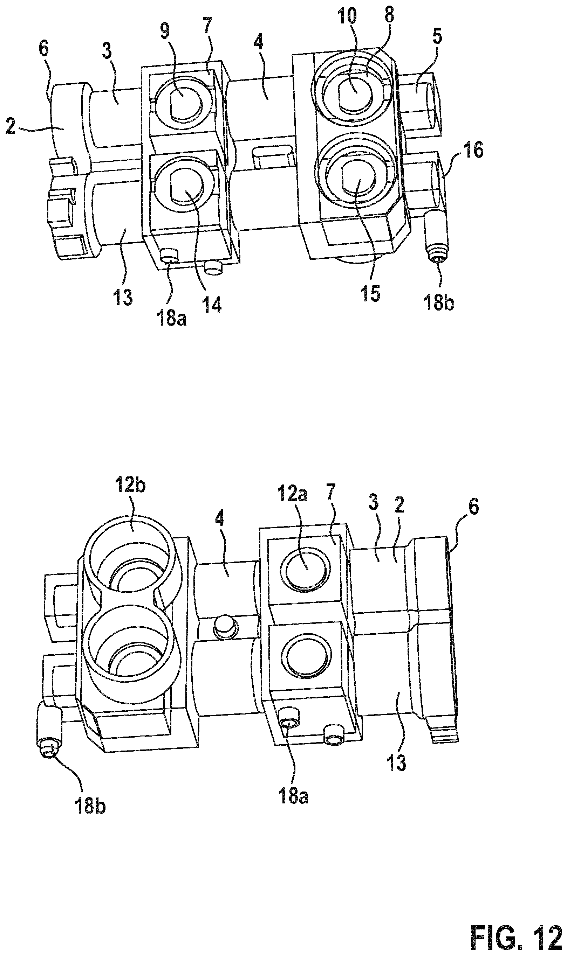

An einer Unterseite der Baugruppe sind Positionierstifte 18a gebildet (vgl. Fig. 12). Die Positionierstifte können beim Anordnen der Baugruppe auf dem Gehäuse des Implantats in zugeordneten Aufnahmen angeordnet werden. In der gezeigten Ausführungsform sind zwei Positionierstifte gezeigt, andere Zahlen von Positionierstiften sind jedoch möglich.

Die erste Aufnahmeeinrichtung 2 und die zweite Aufnahmeeinrichtung 13 weisen jeweils eine Federhülse und eine Steckeraufnahme auf. Die erste Aufnahmeeinrichtung 2 und die zweite Aufnahmeeinrichtung 13 können als IS-1 Konnektoren ausgebildet sein.

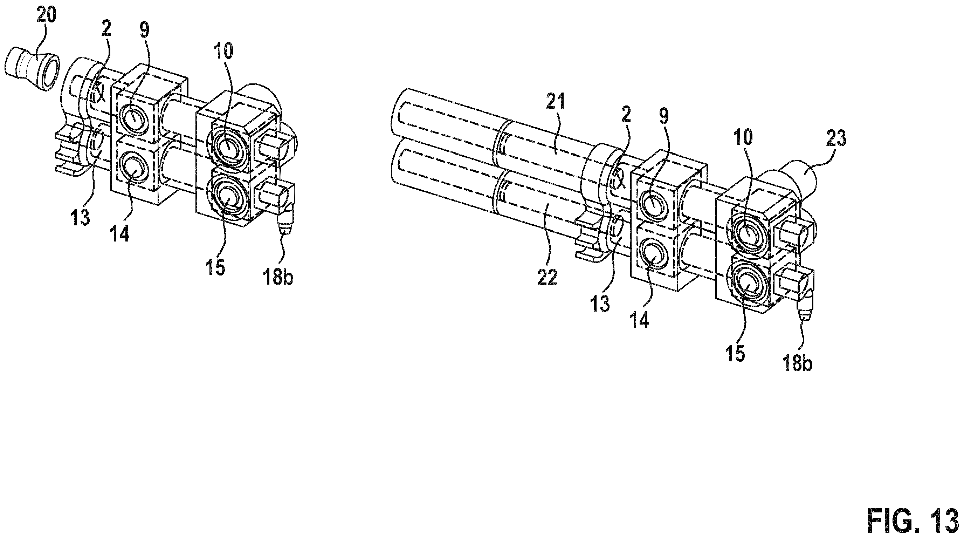

Ein Federelement 20 wird in der ersten Aufnahmeeinrichtung 2 angeordnet und dort befestigt (linke Seite von Fig. 13). Die Aussparung 12a in dem Kunststoff wird genutzt, um das im Inneren der ersten Aufnahmeeinrichtung 2 angeordnete Federelement 20 mittels Widerstandsschweißen in das erste Anschlusselement 7 einzuschweißen. Analog wird ein weiteres Federelement in der zweiten Aufnahmeeinrichtung 13 angeordnet und befestigt (nicht dargestellt). Die Öffnungen der Baugruppe 1 werden anschließend mit Vergusshilfsmitteln 21, 22, 23 verschlossen und abgedichtet (rechte Seite von Fig. 13).

In Fig. 14 ist ein weiterer Montageschritt gezeigt. Ein Drahtband 24 ist an der dritten Kontaktfläche 14 befestigt (z.B. verschweißt). An seinem hinteren Ende weist das Drahtband 24 einen Drahtbandanschluss 25 auf, der mit einem Stiftkontakt einer Durchführung 30 (vgl. Fig. 4) verbindbar ist und beispielsweise auf den Stiftkontakt aufgesteckt werden kann. Weitere Drahtbänder sind mit den anderen Kontaktflächen 9, 10, 15 verbunden.

An der Baugruppe 1 ist eine Antenne 26 befestigt. Die Antenne 26 umgreift den ersten Abschnitt 3 der ersten Aufnahmeeinrichtung 2 teilweise und ist hieran geklippt. In einem Bereich zwischen dem ersten Anschlusselement 7 und dem zweiten Anschlusselement 8 weist die Antenne 26 einen U-förmigen Abschnitt 27 auf. Hierdurch ist eine Griffmulde gebildet, die beispielsweise mit einem automatisierten Greifer verwendet werden kann, um die Baugruppe zu halten und zu transportieren. An einem hinteren Ende der Antenne ist ein Antennenanschluss 28 zur Verbindung mit der Durchführung 30 gebildet.

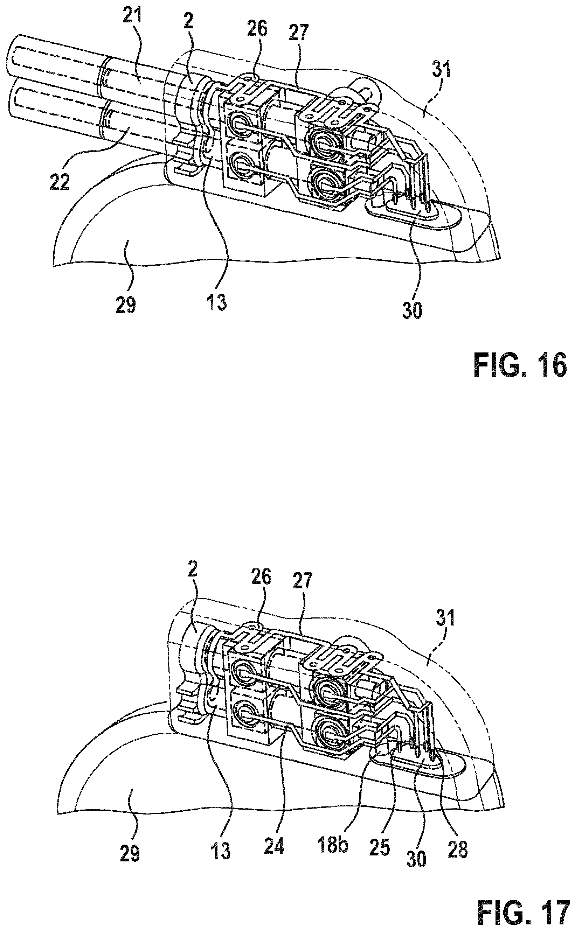

Die Baugruppe mit den Drahtbändern und der Antenne wird anschließend in einer Gießform (z. B. eine Silikonform) angeordnet (nicht dargestellt). Die Drahtbandanschlüsse und der Antennenanschluss 28 werden auf zugeordnete Stifte der Durchführung 30 gesteckt und mit den Stiften verbunden (z. B. verschweißt). Die Gießform wird verschlossen und mit einem Kunstharz 31 (z. B. Epoxidharz) gefüllt. Hierdurch wird die Elektrodenanschlussvorrichtung abgeformt (vgl. Fig. 16).

Die Vergusshilfsmittel 21, 22, 23 werden entfernt und ggf. überschüssiges Harz an den Außenflächen entfernt, z. B. mittels Schleifen und/oder Polieren. Das Implantat mit der Elektrodenanschlussvorrichtung ist hiermit fertig montiert (Fig. 17).

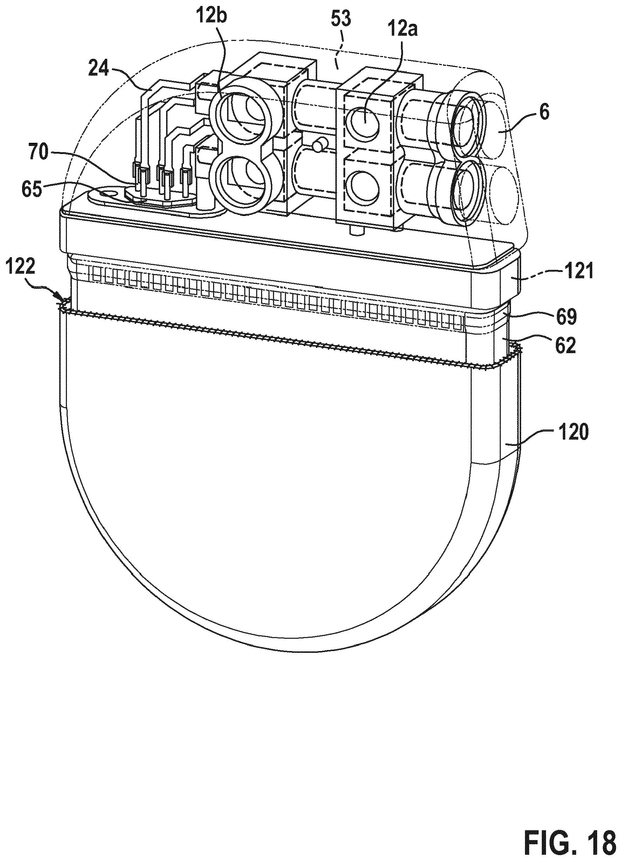

In Fig. 18 ist eine weitere Ausführungsform des Implantats dargestellt. Die Elektrodenanschlussvorrichtung 53 ist an einem Deckel 121 befestigt. Der Energiespeicher 62 ist einem Gehäuse 120 aufgenommen. Das Gehäuse 120 kann als tiefgezogene Form bereitgestellt werden. Der Deckel 121 wird mit dem Gehäuse 120 entlang einer umlaufenden Schweißnaht 122 verschweißt, um das Gehäuse 120 zu schließen. Auf dem Energiespeicher ist ein Stützrahmen 69 angeordnet. Der Stützrahmen 69 nimmt das Elektronikmodul auf. Die Elektrodenanschlussvorrichtung 53 kann beispielsweise gemäß der in den Fig. 11 bis 17 gezeigten Ausführungsform gebildet sein. Die Durchführung 65 mit den Pins 70 ist in den Deckel 121 eingeschweißt.

Die hier offenbarten Ausführungsformen des Implantats und der Verfahren können die folgenden Vorteile aufweisen:
Der Innenaufbau des elektronischen Implantats wird deutlich vereinfacht, so dass Herstellkosten sinken. Ebenso sinkt die Anzahl und Komplexität der benötigten Herstellprozesse, so dass die Skalierbarkeit der Herstellung begünstigt wird (z.B. der vereinfachte Transfer an andere Standorte, geringer Trainingsbedarf für Mitarbeiter, geringere Anforderungen an die benötigte Fertigungsumgebung und begleitendes Engineering). Ferner werden Nacharbeiten möglich oder vereinfacht, wenn lösbare Verbindungstechniken eingesetzt werden.

Mit dem Formfaktor des Elektronikmoduls lässt sich das Implantat verkleinern oder es kann das gewonnene Volumen zur Batteriekapazitätsvergrößerung und damit zur Lebensdauerverlängerung des Implantates verwendet werden. Die Herstellung des Elektronikmoduls in µBGA Technologie ermöglicht eine Steigerung des Nutzens auf dem Panel und eine damit einhergehende Fertigungskostenersparnis. Die vertikale Montage des Elektronikmodules (parallel zur Stirnseite des Energiespeichers) ermöglicht elektrische Verbindungen ohne Winkel und damit einen einfacheren, preisgünstigeren Aufbau des Implantates optimiert für automatische Fertigung in einer Achse.

Die in der Beschreibung, den Ansprüchen und den Figuren offenbarten Merkmale können für die Verwirklichung von Ausführungsformen sowohl einzeln als auch in beliebiger Kombination miteinander relevant sein.

## Patentansprüche

1. Implantat mit einer Elektrodenanschlussvorrichtung und einem Gehäuse, wobei an der Elektrodenanschlussvorrichtung ein Deckel zum Verschluss des Gehäuses gebildet ist.

2. Implantat nach Anspruch 1, wobei der Deckel mit dem Gehäuse verschweißt ist.

3. Implantat nach Anspruch 1 oder 2, wobei an dem Deckel eine Schweißschutzeinrichtung gebildet ist.

4. Implantat nach einem der vorangehenden Ansprüche, wobei der Deckel aus einem biokompatiblen Material gebildet ist.

5. Implantat nach einem der vorangehenden Ansprüche, wobei der Deckel und das Gehäuse aus dem gleichen Material gebildet sind.

6. Implantat nach einem der vorangehenden Ansprüche, wobei im Deckel eine Durchführung gebildet ist, wobei die Durchführung eine elektrische Verbindung zwischen der Elektrodenanschlussvorrichtung und einem in dem Gehäuse angeordneten Elektronikmodul bildet.

7. Implantat nach Anspruch 6, wobei die Durchführung mit einer Steckverbindung oder mit einem Federkontakt mit dem Elektronikmodul elektrisch verbunden ist.

8. Implantat nach einem der vorangehenden Ansprüche, wobei die Elektrodenanschlussvorrichtung eine vormontierte Baugruppe aufweist, wobei die Baugruppe umfasst:
- eine durchgehende Aufnahmeeinrichtung für einen Stecker,
- ein erstes Anschlusselement, das in einem vorderen Bereich der Aufnahmeeinrichtung angeordnet ist, wobei das erste Anschlusselement wenigstens zwei ebene Seitenflächen aufweist, und
- ein zweites Anschlusselement, das in einem hinteren Bereich der Aufnahmeeinrichtung angeordnet ist, wobei das zweite Anschlusselement wenigstens zwei ebene Seitenflächen aufweist.

9. Implantat nach Anspruch 8, wobei die Baugruppe zumindest abschnittsweise von einem Kunststoff umgeben ist.

10. Implantat nach Anspruch 8 oder 9, wobei in dem Kunststoff eine erste Führung für einen ersten Leiter zum Anschluss an einen Anschlussbereich des ersten Anschlusselements gebildet ist und/oder wobei in dem Kunststoff eine zweite Führung für einen zweiten Leiter zum Anschluss an einen Anschlussbereich des zweiten Anschlusselements gebildet ist.

11. Implantat nach Anspruch 10, wobei die erste Führung benachbart zu dem Anschlussbereich des ersten Anschlusselements gebildet ist und/oder wobei die zweite Führung benachbart zu dem Anschlussbereich des zweiten Anschlusselements gebildet ist.

12. Implantat nach einem der Ansprüche 8 bis 11, wobei an einem hinteren Ende der Aufnahmeeinrichtung eine Positioniereinrichtung gebildet ist.

13. Implantat nach einem der Ansprüche 8 bis 11, wobei die Baugruppe des Weiteren eine weitere Aufnahmeeinrichtung für einen weiteren Stecker aufweist, wobei in einem vorderen Bereich der weiteren Aufnahmeeinrichtung ein drittes Anschlusselement angeordnet ist, und wobei in einem hinteren Bereich der weiteren Aufnahmeeinrichtung ein viertes Anschlusselement angeordnet ist.

14. Implantat nach einem der vorangehenden Ansprüche, wobei in der Elektrodenanschlussvorrichtung eine Antenne, eine Ladespule, ein Röntgenmarker, eine Kommunikationsspule und/oder eine Farbmarkierung angeordnet sind.

15. Verfahren zum Herstellen eines Implantats, mit folgenden Schritten:
- Bereitstellen eines Gehäuses,
- Bereitstellen einer Elektrodenanschlussvorrichtung, wobei an der Elektrodenanschlussvorrichtung ein Deckel zum Verschluss des Gehäuses gebildet ist,
- Anordnen des Deckels auf dem Gehäuse und
- Verbinden des Deckels mit dem Gehäuse.
